(19) Europäisches Patentamt / European Patent Office / Office européen des brevets

(11) **EP 4 722 248 A1**

(12) **EUROPEAN PATENT APPLICATION**
published in accordance with Art. 153(4) EPC

(43) Date of publication:
**08.04.2026 Bulletin 2026/15**

(21) Application number: **24815518.6**

(22) Date of filing: **29.05.2024**

(51) International Patent Classification (IPC):
*C07K 16/28* (2006.01)     *A61K 39/395* (2006.01)
*A61P 35/00* (2006.01)     *A61P 35/02* (2006.01)
*A61P 43/00* (2006.01)     *C07K 16/46* (2006.01)
*C12N 15/13* (2006.01)     *C12N 15/63* (2006.01)

(52) Cooperative Patent Classification (CPC):
**A61K 39/395; A61P 35/00; A61P 35/02;
A61P 43/00; C07K 16/00; C07K 16/28;
C07K 16/46; C12N 15/63**

(86) International application number:
**PCT/JP2024/019695**

(87) International publication number:
**WO 2024/248037 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.05.2023 JP 2023088298**

(71) Applicants:
• **Shionogi & Co., Ltd
Osaka-shi, Osaka 541-0045 (JP)**
• **The University of Osaka
Osaka 565-0871 (JP)**

(72) Inventors:
• **YOSHIDA, Tetsuya
Osaka-shi, Osaka 541-0045 (JP)**
• **HAGIWARA, Masaki
Osaka-shi, Osaka 541-0045 (JP)**
• **HARUNA, Miya
Osaka-shi, Osaka 541-0045 (JP)**
• **OHKURA, Naganari
Suita-shi, Osaka 565-0871 (JP)**
• **SAKAGUCHI, Shimon
Suita-shi, Osaka 565-0871 (JP)**
• **WADA, Hisashi
Suita-shi, Osaka 565-0871 (JP)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **BISPECIFIC ANTIBODY THAT RECOGNIZES CCR8 AS ANTIGEN**

(57)     Disclosed is a bispecific antibody that recognizes CCR8 as an antigen. It was found that the bispecific antibody has cytotoxic activity against CCR8-expressing cells.

EP 4 722 248 A1

**Description**

[TECHNICAL FIELD]

The present invention relates to a bispecific antibody that recognizes CCR8 as an antigen and a pharmaceutical composition containing the antibody.

[BACKGROUND ART]

CCR8, also previously called CY6, CKR-L1 or TER1, is a G protein-coupled 7-transmembrane CC chemokine receptor protein expressed in the thymus, the spleen, etc. A gene encoding this protein resides on human chromosome 3p21. Human CCR8 consists of 355 amino acids (Non-patent Document 1). CCL1 is known as an endogenous ligand for CCR8 (Non-patent Document 3). Human CCR8 cDNA is constituted by the nucleotide sequence represented by GenBank ACC No. NM_005201.3, and mouse CCR8 cDNA is constituted by the nucleotide sequence represented by GenBank ACC No. NM_007720.2.

CCR8 is specifically expressed in tumor-infiltrating Treg cells, and it has been demonstrated that, when breast cancer cells were inoculated in CCR8-deficient and wild-type mice, breast cancer proliferation and metastasis were more suppressed in the CCR8-deficient mouse compared to the wild-type mice (Patent Document 1 and Non-patent Document 3). Non-patent Documents 4 to 8 also describe that CCR8 is involved in the pathology of cancer. Furthermore, it has been disclosed that anti-CCR8 antibody administration to a cancer model animal showed an anti-tumor effect (Patent Documents 2 to 24 and 31 to 48).

It has also been disclosed that tumor cells themselves are expressing CCR8 in many blood cancers and melanoma (Patent Documents 25 and 26, Non-patent Documents 9 to 11). However, a treatment effect of anti-CCR8 antibody against cancer expressing CCR8 in tumor cells has not specifically been shown so far as experimental results.

A bispecific antibody that recognizes a surface maker of a tumor cell and a surface marker of an effector cell is known to be useful for cancer treatment (Non-patent Document 12). Effector cells exhibit strong cytotoxic activity against target cells by the bispecific antibody linking target cells with effector cells. Examples of the bispecific antibodies that recognizes CD3 that is a surface marker of T cells is disclosed in Patent Documents 27 to 29. Also, Examples of the bispecific antibodies that recognizes CD16 that is a surface marker of NK cells are disclosed in Patent Documents 27 to 29.

However, it has not been shown so far that a bispecific antibody that recognizes CCR8 as an antigen is useful for cancer treatment. Also, it has not been shown so far that the bispecific antibody, that recognizes surface markers of tumor-infiltrating Treg cells and effector cells, is useful for cancer treatment. The bispecific antibody, that recognizes CCR8 as an antigen, has been shown in Patent Document 20, but no pharmacological activity of the bispecific antibody has been disclosed.

[PRIOR ART REFERENCES]

[Patent Documents]

[Patent Document 1] US 10087259
[Patent Document 2] WO 2018/181425
[Patent Document 3] WO 2018/112032
[Patent Document 4] WO 2020/138489
[Patent Document 5] WO 2021/142002
[Patent Document 6] WO 2021/152186
[Patent Document 7] WO 2021/163064
[Patent Document 8] WO 2021/178749
[Patent Document 9] WO 2021/194942
[Patent Document 10] WO 2021/260208
[Patent Document 11] WO 2021/260209
[Patent Document 12] WO 2021/260210
[Patent Document 13] WO 2022/042690
[Patent Document 14] WO 2022/078277
[Patent Document 15] WO 2022/081718
[Patent Document 16] WO 2022/117569
[Patent Document 17] WO 2022/136647

[Patent Document 18] WO 2022/136649
[Patent Document 19] WO 2022/136650
[Patent Document 20] WO 2022/256563
[Patent Document 21] WO 2022/268192
[Patent Document 22] WO 2023/288241
[Patent Document 23] WO 2023/010054
[Patent Document 24] WO 2023/020621
[Patent Document 25] WO 2022/200303
[Patent Document 26] WO 2013/131010
[Patent Document 27] WO 99/54440
[Patent Document 28] WO 2016/182751
[Patent Document 29] WO 2017/134158
[Patent Document 30] WO 2020/081841
[Patent Document 31] WO 2023/098888
[Patent Document 32] WO 2023/116880
[Patent Document 33] WO 2023/137466
[Patent Document 34] WO 2023/174396
[Patent Document 35] WO 2023/193732
[Patent Document 36] WO 2023/206350
[Patent Document 37] WO 2023/208182
[Patent Document 38] WO 2024/008110
[Patent Document 39] WO 2024/040216
[Patent Document 40] WO 2024/059909
[Patent Document 41] WO 2024/062019
[Patent Document 42] WO 2024/062072
[Patent Document 43] WO 2024/062076
[Patent Document 44] WO 2024/062082
[Patent Document 45] WO 2024/076514
[Patent Document 46] WO 2024/086684
[Patent Document 47] WO 2024/088346
[Patent Document 48] WO 2024/094003

[Non-patent Documents]

[Non-patent Document 1] J. Immunol., 1996, Vol .157, No. 7, p.2759-63
[Non-patent Document 2] J. Biol. Chem.,1997, Vol. 272, No. 28, p.17251-4
[Non-patent Document 3] Cancer Res.,2016, Vol. 76, No. 4, Supp.1, P4-04-11
[Non-patent Document 4] Immunity,2016, Vol. 45, p.1122-1134
[Non-patent Document 5] Immunity,2016, Vol. 45, p.1135-1147
[Non-patent Document 6] Proc. Natl. Acad. Sci. USA, 2018, Vol. 115, No. 45, p.10672-10681
[Non-patent Document 7] Targeting of CCR8 induces anti-tumor activity as a monotherapy that is further enhanced in combination with a Listeria-based immunotherapy, ASCO 2018, Daniel O Villarreal, et al. https://www.advaxis.com/wp-content/uploads/2018/04/KeystonePosterCCR8-combo-posterFINAL.pdf
[Non-patent Document 8] Cancer Res., 2018, Vol. 78, No. 18, p.5340-5348
[Non-patent Document 9] Leuk. Lymphoma., 2006, Vol. 47, No. 10, p.2163-73
[Non-patent Document 10] Appl. Immunohistochem. Mol. Morphol., 2015, Vol. 23, No. 8, p.580-589
[Non-patent Document 11] Modern Pathology, 2013, Vol. 26, p.182-194
[Non-patent Document 12] Exp. Hematol., 2021, 10:56

[SUMMARY OF THE INVENTION]

[PROBLEMS TO BE SOLVED BY THE INVENTION]

An object of the present invention is to provide a bispecific antibody that recognizes CCR8 as an antigen. A further object of the present invention is to provide the bispecific antibody that recognizes CCR8 as an antigen, which is useful for cancer treatment and prevention of cancer.

[MEANS FOR SOLVING THE PROBLEMS]

The present inventors have found, as a result of intensive studies, a bispecific antibody that recognizes CCR8 as an antigen. The present inventors have further found that the bispecific antibody that recognizes CCR8 as an antigen of the present invention has an anti-tumor effect. In other words, the bispecific antibody that recognizes CCR8 as an antigen of the present invention is useful for cancer treatment and prevention of cancer.

Specifically, the present invention relates to the following.

(1) A bispecific antibody that comprises an anti-CCR8-scFv region comprising a light chain variable region and a heavy chain variable region of an anti-CCR8 antibody and a region comprising one or more of a light chain variable region or a heavy chain variable region of an antibody that recognizes a surface marker of an effector cell.

(2) The bispecific antibody according to (1), wherein the anti-CCR8-scFv region comprises:

1) a light chain variable region including

CDR1 having the amino acid sequence of SEQ ID NO: 57,
CDR2 having the amino acid sequence of SEQ ID NO: 58, and
CDR3 having the amino acid sequence of SEQ ID NO: 59, and
a heavy chain variable region including
CDR1 having the amino acid sequence of SEQ ID NO: 60,
CDR2 having the amino acid sequence of SEQ ID NO: 61, and
CDR3 having the amino acid sequence of SEQ ID NO: 62;

2) a light chain variable region including

CDR1 having the amino acid sequence of SEQ ID NO: 57,
CDR2 having the amino acid sequence of SEQ ID NO: 63, and
CDR3 having the amino acid sequence of SEQ ID NO: 59, and
a heavy chain variable region including
CDR1 having the amino acid sequence of SEQ ID NO: 64,
CDR2 having the amino acid sequence of SEQ ID NO: 61, and
CDR3 having the amino acid sequence of SEQ ID NO: 65;

3) a light chain variable region including

CDR1 having the amino acid sequence of SEQ ID NO: 66,
CDR2 having the amino acid sequence of SEQ ID NO: 67, and
CDR3 having the amino acid sequence of SEQ ID NO: 68, and
a heavy chain variable region including
CDR1 having the amino acid sequence of SEQ ID NO: 69,
CDR2 having the amino acid sequence of SEQ ID NO: 70, and
CDR3 having the amino acid sequence of SEQ ID NO: 71;

4) a light chain variable region including

CDR1 having the amino acid sequence of SEQ ID NO: 72,
CDR2 having the amino acid sequence of SEQ ID NO: 67, and
CDR3 having the amino acid sequence of SEQ ID NO: 73, and
a heavy chain variable region including
CDR1 having the amino acid sequence of SEQ ID NO: 74,
CDR2 having the amino acid sequence of SEQ ID NO: 75, and
CDR3 having the amino acid sequence of SEQ ID NO: 76; or

5) a light chain variable region including

CDR1 having the amino acid sequence of SEQ ID NO: 77,
CDR2 having the amino acid sequence of SEQ ID NO: 78, and
CDR3 having the amino acid sequence of SEQ ID NO: 79, and

a heavy chain variable region including
CDR1 having the amino acid sequence of SEQ ID NO: 80,
CDR2 having the amino acid sequence of SEQ ID NO: 81, and
CDR3 having the amino acid sequence of SEQ ID NO: 82.

(3) The bispecific antibody according to (1), wherein the anti-CCR8-scFv region comprises:

1) a light chain variable region having the amino acid sequence of SEQ ID NO: 45, and
a heavy chain variable region having the amino acid sequence of SEQ ID NO: 46;
2) a light chain variable region having the amino acid sequence of SEQ ID NO: 47, and
a heavy chain variable region having the amino acid sequence of SEQ ID NO: 48;
3) a light chain variable region having the amino acid sequence of SEQ ID NO: 49, and
a heavy chain variable region having the amino acid sequence of SEQ ID NO: 50;
4) a light chain variable region having the amino acid sequence of SEQ ID NO: 51, and
a heavy chain variable region having the amino acid sequence of SEQ ID NO: 52;
5) a light chain variable region having the amino acid sequence of SEQ ID NO: 53, and
a heavy chain variable region having the amino acid sequence of SEQ ID NO: 54; or
6) a light chain variable region having the amino acid sequence of SEQ ID NO: 55, and
a heavy chain variable region having the amino acid sequence of SEQ ID NO: 56.

(4) The bispecific antibody according to any one of (1) to (3), wherein the light chain variable region and the heavy chain variable region are linked via the linker sequence of SEQ ID NO: 2 in the anti-CCR8-scFv region.
(5) The bispecific antibody according to any one of (1) to (4), wherein a CD8A signal sequence region is linked to an N-terminal.
(6) The bispecific antibody according to (5) wherein the CD8A signal sequence region is present on a N-terminal side of the anti-CCR8-scFv region.
(7) The bispecific antibody according to (5) or (6), wherein the CD8A signal sequence region that has the amino acid sequence of SEQ ID NO: 1.
(8) The bispecific antibody according to any one of (1) to (7), wherein the anti-CCR8-scFv region has the amino acid sequence of any one of SEQ ID NOs: 39 to 44.
(9) The bispecific antibody according to any one of (1) to (8), wherein the effector cell is a T cell.
(10) The bispecific antibody according to (9), wherein the surface marker of the T cell is CD3, comprising an anti-CD3-scFv region that comprises the light chain variable region and the heavy chain variable region of an antibody that recognizes CD3.
(11) The bispecific antibody according to (10), wherein the anti-CD3-scFv region comprises:

1) a light chain variable region including

CDR1 having the amino acid sequence of SEQ ID NO: 16,
CDR2 having the amino acid sequence of SEQ ID NO: 17, and
CDR3 having the amino acid sequence of SEQ ID NO: 18, and
a heavy chain variable region including
CDR1 having the amino acid sequence of SEQ ID NO: 19,
CDR2 having the amino acid sequence of SEQ ID NO: 20, and
CDR3 having the amino acid sequence of SEQ ID NO: 21;

2) a light chain variable region including

CDR1 having the amino acid sequence of SEQ ID NO: 22,
CDR2 having the amino acid sequence of SEQ ID NO: 23, and
CDR3 having the amino acid sequence of SEQ ID NO: 24, and
a heavy chain variable region including
CDR1 having the amino acid sequence of SEQ ID NO: 25,
CDR2 having the amino acid sequence of SEQ ID NO: 26, and
CDR3 having the amino acid sequence of SEQ ID NO: 27;

3) a light chain variable region including

CDR1 having the amino acid sequence of SEQ ID NO: 28,
CDR2 having the amino acid sequence of SEQ ID NO: 29, and
CDR3 having the amino acid sequence2 of SEQ ID NO: 30, and
a heavy chain variable region including
CDR1 having the amino acid sequence of SEQ ID NO: 31,
CDR2 having the amino acid sequence of SEQ ID NO: 32, and
CDR3 having the amino acid sequence of SEQ ID NO: 33; or

4) a light chain variable region including

CDR1 having the amino acid sequence of SEQ ID NO: 87,
CDR2 having the amino acid sequence of SEQ ID NO: 88, and
CDR3 having the amino acid sequence of SEQ ID NO: 89, and
a heavy chain variable region including
CDR1 having the amino acid sequence of SEQ ID NO: 90,
CDR2 having the amino acid sequence of SEQ ID NO: 91, and
CDR3 having the amino acid sequence of SEQ ID NO: 92.

(12) The bispecific antibody according to (10), wherein the anti-CD3-scFv region comprises:

1) a light chain variable region having the amino acid sequence of SEQ ID NO: 10, and
a heavy chain variable region having the amino acid sequence of SEQ ID NO: 11;
2) a light chain variable region having the amino acid sequence of SEQ ID NO: 12, and
a heavy chain variable region having the amino acid sequence of SEQ ID NO: 13;
3) a light chain variable region having the amino acid sequence of SEQ ID NO: 14, and
a heavy chain variable region having the amino acid sequence of SEQ ID NO: 15; or
4) a light chain variable region having the amino acid sequence of SEQ ID NO: 85, and
a heavy chain variable region having the amino acid sequence of SEQ ID NO: 86.

(13) The bispecific antibody according to (10), wherein the anti-CD3-scFv region has the amino acid sequence of SEQ ID NO: 7, 8, 9 or 84.
(14) The bispecific antibody according to (9), wherein the surface marker of the T cell is CD3, comprising an anti-CD3-VHH region comprises a heavy chain variable region of a VHH antibody that recognizes CD3.
(15) The bispecific antibody according to any one of (1) to (8), wherein the effector cell is an NK cell.
(16) The bispecific antibody according to (15), wherein the surface marker of the NK cell is CD16, comprising an anti-CD16-scFv region that comprises a light chain variable region and a heavy chain variable region of an antibody that recognizes CD16.
(17) The bispecific antibody according to (15), wherein the surface marker of the NK cell is CD16, comprising an anti-CD16-VHH region that comprises a heavy chain variable region of a VHH antibody that recognizes CD16.
(18) The bispecific antibody according to claim 15, wherein the surface marker of the NK cell is CD16, comprising an anti-CD16-VHH region that comprises a heavy chain variable region of a VHH antibody recognizing CD16.
(18) The bispecific antibody according to (17), wherein the anti-CD16-VHH region comprises a heavy chain variable region including

CDR1 having the amino acid sequence of SEQ ID NO: 35,
CDR2 having the amino acid sequence of SEQ ID NO: 36, and
CDR3 having the amino acid sequence of SEQ ID NO: 37.

(19) The bispecific antibody according to (17), wherein the anti-CD16-VHH region has the amino acid sequence of SEQ ID NO: 34.
(20) The bispecific antibody according to any one of clams (1) to (19), wherein the anti-CCR8-scFv region that comprising the light chain variable region and the heavy chain variable region of the anti-CCR8 antibody and the region comprising one or more of a light chain variable region or a heavy chain variable region of the antibody that recognizes the surface maker of the effector cell are linked via an scFv linker sequence of any one of SEQ ID NOs: 3 to 6.
(21) The bispecific antibody according to any one of (1) to (20), wherein a histidine tag having the amino acid sequence of SEQ ID NO: 38 is linked to a C-terminal.
(22) The bispecific antibody according to any one of (1) to (21) having a Fc region.

(23) A pharmaceutical composition comprising the bispecific antibody according to any one of (1) to (22).

(24) The pharmaceutical composition according to (23), for use in treating cancer.

(25) The pharmaceutical composition according to (24) having an effect of depleting tumor-infiltrating Treg cells expressing CCR8.

(26) The pharmaceutical composition according to (24), for use in treating cancer not expressing CCR8 in tumor cells.

(27) The pharmaceutical composition according to (24), for use in treating cancer expressing CCR8 in tumor cells.

(28) A polynucleotide encoding an amino acid sequence of the bispecific antibody according to any one of (1) to (22).

(29) An expression vector comprising the polynucleotide according to (28).

[EFFECTS OF THE INVENTION]

Since the bispecific antibody that recognizes CCR8 as an antigen of the present invention has an anti-tumor effect, the bispecific antibody is very useful as a medicament, particularly as a medicament for the treatment and prevention of cancer.

[BRIEF DESCRIPTION OF DRAWINGS]

Figure 1 shows cytotoxic activity of an CCR8-CD3 bispecific antibody against human CCR8-expressing cells using PBMCs as effector cells. It was evaluated using human CCR8-expressing Rat-1 cells (hCCR8/Rat-1 cells) as target cells.

Figure 2 shows cytotoxic activity of the CCR8-CD3 bispecific antibodies against human CCR8-expressing cells using unstimulated PBMCs as effector cells. It was evaluated using firefly luciferase expressing hCCR8/Rat-1 cells (hCCR8/FLuc/Rat-1 cells) as target cells.

Figure 3 shows cytotoxic activity of CCR8-CD3 bispecific antibodies against human CCR8-expressing cells using CD8-positive T cells as effector cells. It was evaluated using hCCR8/FLuc/Rat-1 cells as target cells.

Figure 4 shows analysis of Foxp3 and CCR8 expression in an adult T-cell leukemia lymphoma (ATLL) cell line. ATN-1 cells were used as the ATLL cell line.

Figure 5 shows cytotoxic activity of CCR8-CD3 bispecific antibodies against ATLL cell line using CD8-positive T cells as effector cells. It was evaluated using firefly luciferase expressing ATN-1cells (FLuc/ATN-1 cells) as effector cells. In Figure 5, "B1", "B2", "B3", "B4", "B5", and "B6" mean B1-CD3-1, B2-CD3-1, B3-CD3-1, B4-CD3-1, B5-CD3-1, and B6-CD3-1 of the CCR8-CD3 bispecific antibodies, respectively.

Figure 6 shows concentration-dependent cytotoxic activity of CCR8-CD3 bispecific antibodies against ATLL cell line using CD8-positive T cells as effector cells. It was evaluated using FLuc/ATN-1 cells as target cells.

Figure 7 shows NFAT activation potency of CCR8-CD3 bispecific antibodies using Jurkat cells as effector cells. It was evaluated using FLuc/ATN-1 cells as target cells. The Jurkat cells, since a Lucia luciferase gene-linked gene was introduced into downstream of an NFAT promoter, expressed Lucia luciferase by NFAT activation.

Figure 8 shows cytotoxic activity of CCR8-CD3 bispecific antibodies, wherein an anti-CD3-scFv region is different, against human CCR8-expressing cells using PBMCs as effector cells. It was evaluated using hCCR8/FLuc/Rat-1 cells as target cells.

Figure 9 shows cytotoxic activity of CCR8-CD3 bispecific antibodies, wherein length of an scFv linker is different, against human CCR8-expressing cells using PBMCs as effector cells. It was evaluated using hCCR8/FLuc/Rat-1 cells as target cells.

Figure 10 shows cytotoxic activity of CCR8-CD3 bispecific antibodies against mature Treg cells using CD8-positive T cells as effector cells. It was evaluated using the mature Treg cells that were obtained by carrying out expansion culture of naive Treg cells separated from human PBMCs, as target cells. In Figure 10, "B5" and "B6" mean B5-CD3-1 and B6-CD3-1 of the CCR8-CD3 bispecific antibodies, respectably.

Figure 11 shows cytotoxic activity of CCR8-CD3 bispecific antibodies against human tumor-infiltrating Treg cells using CD8-positive T cells as effector cells. It was evaluated by using tumor-infiltrating cells derived from ovarian cancer patients as target cells, recovering cells after an assay and conducting a flow cytometry analysis. In Figure 11, "B5" and "B6" mean B5-CD3-1 and B6-CD3-1 of the CCR8-CD3 bispecific antibodies, respectably.

Figure 12 shows cytotoxic activity of CCR8-CD3 bispecific antibodies against CD8-positive T cells using CD8-positive T cells as effector cells. It was evaluated by using tumor-infiltrating cells derived from ovarian cancer patients as target cells, recovering the cells after an assay and conducting a flow cytometry analysis. In Figure 11, "B5" and "B6" mean B5-CD3-1 and B6-CD3-1 of the CCR8-CD3 bispecific antibodies, respectably.

Figure 13 shows cytotoxic activity of CCR8-CD3 bispecific antibodies against CD4-positive Tconv cells using the CD8-positive T cells as effector cells. It was evaluated by using tumor-infiltrating cells derived from ovarian cancer patients as target cells, recovering the cells after an assay and conducting a flow cytometry analysis. In Figure 11, "B5" and "B6" mean B5-CD3-1 and B6-CD3-1 of the CCR8-CD3 bispecific antibodies, respectably.

Figure 14 shows cytotoxic activity of CCR8-CD16 bispecific antibodies against human CCR8-expressing cells using human peripheral blood-derived NK cells as effector cells. It was evaluated hCC8/FLuc/Rat-1 cells as target cells. In Figure 14, "B1", "B2", "B3", "B4", "B5", and "B6" mean B1-CD16-VHH, B2- CD16-VHH, B3- CD16-VHH, B4- CD16-VHH, B5- CD16-VHH, and B6- CD16-VHH of the CCR8-CD16 bispecific antibodies, respectably.

Figure 15 shows cytotoxic activity of CCR8-CCR16 bispecific antibody against ATLL cell line using KHYG-1 cells, a human NK cell line, as effector cells. It was evaluated using ATN-1 cells as target cells.

Figure 16 shows cytotoxic activity of CCR8-CD16 bispecific antibody against mature Treg cells using KHYG-1 cells, a human NK cell line, as effector cells. It was evaluated using the mature Treg cells that were obtained by carrying out expansion culture of naive Treg cells separated from human PBMCs, as target cells.

Figure 17 shows cytotoxic activity against hCCR8/Rat-1 cells and CCR8-non-expressing Rat-1 cells using KHYG-1 cells that are human NK cell lines as effector cells.

Figure 18-1 shows cytotoxic activity of CCR8-CD3 bispecific antibody comprising an anti-mouse CD3-scFv region against human CCR8 expressing cells using mouse spleen-derived CD8-positive T cells as effector cells. It was evaluated using hCCR8/FLuc/Rat-1 cells as target cells.

Figure 18-2 shows cytotoxic activity of a CCR8-CD3 bispecific antibody comprising an anti-mouse CD3-scFv region against human CCR8 expressing cells using mouse spleen-derived CD8-positive T cells as effector cells. It was evaluated using hCCR8/FLuc/Rat-1 cells as target cells. It shows specific activity when a maximum killing rate is taken as 100 %.

Figure 19 shows an anti-tumor activity of a CCR8-CD3 bispecific antibody comprising an anti-mouse CD3-scFv region in mice inoculated with hCCR8/Fluc/Rat-1 cells using mouse spleen-derived CD8-positive T cells as effector cells. Luminescence intensity reflects the number of hCCR8/Fluc/Rat-1 cells.

Figure 20 shows anti-tumor activity in mice inoculated with hCCR8/Fluc/Rat-1 cells to which PBMCs and CCR8-CD3 bispecific antibody were intraperitoneally administered by luminescence image data.

Figure 21 shows an anti-tumor activity of the CCR8-CD3 bispecific antibody in mice inoculated with hCCR8/Fluc/Rat-1 cells using PBMCs as effector cells. The luminescence intensity reflects the number of hCCR8/Fluc/Rat-1 cells.

Figure 22 shows an anti-tumor activity in mice inoculated with FLuc/ATN-1 cells to which PBMCs and a CCR8-CD3 bispecific antibody were intraperitoneally administered by luminescence image data.

Figure23-1 shows an anti-tumor activity of a CCR8-CD3 bispecific antibody in mice inoculated with FLuc/ ATN-1 cells using PBMCs as effector cells. The luminescence intensity reflects the number of FLuc/ATN-1 cells.

Figure23-2 shows an anti-tumor activity of a CCR8-CD3 bispecific antibody in mice inoculated with FLuc/ ATN-1 cells using PBMCs as effector cells. The luminescence intensity reflects the number of FLuc/ATN-1 cells.

Figure24 shows cytotoxic activity of CCR8-CD3 bispecific antibody and CD3-CCR8 bispecific antibody against ATLL cell lines using PBMCs as effector cells.

Figure 25 shows cytotoxic activity of CCR8-CD3 bispecific antibodies, wherein length of an scFv linker is different, against human CCR8-expressing cells using PBMCs as effector cells. It was evaluated using the hCCR8/FLuc/Rat-1 cells as target cells.

Figure 26 shows cytotoxic activity of a CCR8-CD3 bispecific antibody to which a Fc region is added against human CCR8-expressing cells using PBMCs as effector cells. It was evaluated using hCCR8/FLuc/Rat-1 cells as target cells.

Figure 27 shows cytotoxic activity of a CCR8-CD3 bispecific antibody to which a Fc region is added against ATLL cell lines using PBMCs as effector cells. It was evaluated using FLuc/ATN-1 cells as target cells.

Figure 28 shows a comparison of NF-AT promoter induction activity with the CCR8-CD3 bispecific antibody based on Patent Document 20.

Figure 29 shows a comparison of cytotoxic activity against hCCR8/FLuc/Rat-1 cells with the CCR8-CD3 bispecific antibody based on Patent Document 20.

Figure 30 shows a comparison of cytotoxic activity against FLuc/ATN-1 cells with the CCR8-CD3 bispecific antibody based on Patent Document 20.

## [MODE FOR CARRYING OUT THE INVENTION]

The term used in the present description is used in the meaning usually used in the art, unless specifically mentioned.

The bispecific antibody of the present invention comprises an anti-CCR8-scFv region comprising a light chain variable region and a heavy chain variable region of an anti-CCR8 antibody and a region comprising one or more of a light chain variable region or a heavy chain variable region of an antibody that recognizes a surface marker of an effector cell.

The bispecific antibody of the present invention can be obtained by determining an entire amino acid sequence on the basis of amino acid sequence information on an anti-CCR8-scFv region comprising a light chain variable region and a heavy chain variable region of an anti-CCR8 antibody and a region comprising one or more of a light chain variable region or a heavy chain variable region of an antibody binding to a surface marker of an effector cell to construct an expression vector, and being expressed in cells or the like using the expression vector. Any promoter may also be used in the expression vector.

Examples of cells expressing the bispecific antibody of the precent invention include a vertebrate cell such a COS cell, a CHO cell, a 293 cell, or a 293T cell, a prokaryotic cell, and a yeast. A transformant can be cultured according to a method well known to those skilled in the art, and the bispecific antibody of the present invention is produced intracellularly or extracellularly of the transformant. The medium used for the culture can be selected as appropriate depending on the adopted host cell from various types of media commonly used. For example, examples of the medium include a medium such as RPMI-1640 medium or Dulbecco's Modified Eagle Minimum Essential Medium (DMEM) supplemented with serum components such as fetal calf serum (FCS), as necessary. The medium may be supplemented with antibiotics such as penicillin, streptomycin, and kanamycin, as necessary. Also, the medium may be supplemented with antibiotics such as geneticin, hygromycin, or puromycin for drug selection, as necessary. The culture temperature during culture of the transformant may be any temperature that does not significantly reduce protein synthesis capacity in the cell, and is preferably 32 to 42°C, most preferably 37°C. As necessary, the transformant can be cultured in an atmosphere containing 1 to 10% (v/v) of carbon dioxide.

Fractions comprising the bispecific antibody of the present invention produced intracellularly or extracellularly of the transformant as described above can be separated and purified by various known separation methods utilizing the physical and chemical properties or the like of the proteins. Specific examples of such methods include usual treatment with a protein precipitant, ultrafiltration, various chromatography such as molecular sieve chromatography (gel filtration), adsorption chromatography, ion exchange chromatography, affinity chromatography, or high-performance liquid chromatography (HPLC), a dialysis method, and a combination of these. With the methods, the bispecific antibody of the present invention can be readily produced in high yield and high purity.

An anti-CCR8-scFv region comprising a light chain variable region and a heavy chain variable region of an anti-CCR8 antibody means a region that can specifically bind to CCR8 similarly to the antibody and that is an scFV fragment derived from an anti-CCR8 antibody.

A region comprising one or more of a light chain variable region or a heavy chain variable region of an antibody that recognizes a surface marker of an effector cell means a region that can specifically bind to a surface marker of an effector cell similarly to the antibody and that is a fragment derived from the antibody binding to a surface marker of an effector cell. Examples of the region include fragments of scFv and VHH but these are not particularly limited.

scFv is a VH-P-VL or VL-P-VH polypeptide in which one heavy chain variable region (VH) and one light chain variable region (VL) are linked with an appropriate linker sequence (hereinafter referred to as P). A VH-P-VL polypeptide is preferred. The VH and VL contained in the scFv used in the present invention may be those of the antibody according to the present invention. The scFv used in the present invention can also be produced by constructing an scFv expression vector using cDNA encoding the VH and VL of the antibody according to the present invention and introducing the vector into E. coli, yeast, or an animal cell to express the scFv.

VHH is a heavy chain variable region in an antibody (VHH antibody) comprising only a heavy chain obtained from animals of Camelidae animals such as llama, camel, or alpaca and also called nanobody. The VHH used in the present invention can also be produced by constructing a VHH expression vector using cDNA encoding the VHH of the antibody according to the present invention and introducing the vector into E. coli, yeast, or an animal cell to express the VHH.

A peptide comprising a CDR, such as scFv and VHH, is composed of at least one or more regions of a CDR of VH or VL. The plurality of CDRs can be bound directly or via an appropriate linker sequence. The peptide comprising the CDR used in the present invention can be produced by constructing a CDR-encoding DNA using cDNA encoding the VH and VL of a monoclonal antibody of the present invention, inserting the DNA into an animal cell expression vector, and introducing the vector into E. coli, yeast, or an animal cell to express the peptide. The peptide comprising a CDR can also be produced by chemical synthesis methods such as the Fmoc method (fluorenylmethyloxycarbonyl method), the tBoc method (t-butyloxycarbonyl method), and the like.

Specific binding can be characterized by an equilibrium dissociation constant of at least about $1 \times 10^{-6}$ M or less (for example, the smaller Kd represents the closer binding). The Kd value is preferably $1 \times 10^{-7}$ M or less, more preferably $1 \times 10^{-8}$ M or less, still more preferably $1 \times 10^{-9}$ M or less. Methods for determining whether two molecules specifically bind are well known in the art, and examples thereof include competitive ELISA methods, surface plasmon resonance, and the like other method.

Antibody-producing techniques known in the art can be used for an anti-CCR8 antibody. Examples of the techniques include a method described in Immunochemistry in Practice (Blackwell Scientific Publiations).

Genetically engineered techniques known in the art can also be used. Examples of the techniques include methods described in Molecular Cloning, A Laboratory Manual, Fourth Edition, Cold Spring Harbor Laboratory Press (2012), and Current Protocols Essential Laboratory Techniques, Current Protocols (2012).

The amino acid sequence of human CCR8 is shown in UniProtKB/Swiss-Prot: P51685 (SEQ ID NO: 83). The extramembrane domains of human CCR8 correspond to the N-terminal region consisting of amino acids positions 1-35, the loop1 region consisting of amino acids positions 94-107, the loop2 region consisting of amino acids positions 172-202, and the loop3 region consisting of amino acids positions 264-280.

A hybridoma producing an anti-CCR8 antibody can be produced by using, as an immunogen, a human CCR8 protein, a gene encoding the full length of human CCR8, a human CCR8-expressing cell, or the like. When a gene encoding the full length of human CCR8 is used an immunogen, the hybridoma producing the anti-CCR8 antibody can be prepared, for example, by fusing a spleen cell of a mouse which has been DNA-immunized with the gene as an antigen into a mouse myeloma cell.

One aspect of an anti-CCR8 antibody includes a monoclonal antibody having a CDR or a heavy/light chain variable region described in the present description. The antibody or an antibody fragment may be from any class or subclass of immunoglobulin molecule (e.g., IgG, IgE, IgM, IgD, or IgA, preferably, IgG). The antibody or antibody fragment may also be obtained from any species, such as mouse, rat, shark, rabbit, pig, hamster, camel, llama, goat, or human. The antibody or antibody fragment thereof is preferably a mouse-derived monoclonal antibody or a humanized monoclonal antibody.

An anti-CCR8 antibody is characterized in that it inhibits a binding of CCR8 to a CCR8 ligand. The "CCR8 ligand" is not particularly limited as long as it is a substance that binds to CCR8, such as CCL1, CCL8, or CCL18, but is preferably CCL1, CCL18, and particularly preferably CCL1.

Inhibitory ability of binding of CCR8 to a CCR8 ligand can be determined, when the CCR8 ligand is human CCL1, for example, by using human CCR8-expressing 293 cells, determining $Ca^{2+}$ influx by human CCL1 addition, and calculating an IC50 value with setting that a signal when human CCL1 is not added as the inhibition rate of 100% and the signal when human CCL1 is added and the antibody is not added as the inhibition rate of 0%. The inhibitory ability of binding to other CCR8 ligands can also be determined in a similar manner to human CCL1 as described above.

Human CCL1 has the amino acid sequence shown by UniProtKB/Swiss-Prot No. P22362 or the like. Human CCL8 has the amino acid sequence shown by GenBank No. AAI 26243.1 or the like. Human CCL18 has the amino acid sequence shown by GenBank No. EAW80102.1 or the like.

An anti-CCR8 antibody also include a chimeric antibody, a humanized antibody, and a fully human antibody. The humanized monoclonal antibody is useful when administered to humans for therapeutic purposes or the like, because it is less antigenic in humans. A humanized monoclonal antibody is an antibody in which a complementarity determining region (CDR) of a non-human mammal antibody, such as a mouse antibody, is inserted into a framework region (FR) of a human antibody. The FR of a humanized monoclonal antibody is thus derived from a human. Suitable FR can be selected by referring to the documents of Kabat E.A. et al. As the FR in this case, one with which the CDR can form an appropriate antigen binding site is selected. As necessary, amino acids of the FR of a variable region of the antibody may be substituted so that the CDR of the reconstituted humanized monoclonal antibody form an appropriate antigen binding site (Sato, K. et al., Cancer Res. 1993, vol. 53, p. 851). The percentage of amino acids of the FR to be substituted is from 0 to 15%, preferably from 0 to 5%, of the total FR region.

An anti-CCR8 antibody is preferably a CCR8-neutralizing antibody. The CCR8-neutralizing antibody means an antibody having neutralizing activity against CCR8. Whether or not the antibody has neutralizing activity against CCR8 can be determined, for example, by measuring whether or not the antibody inhibits the physiological action of a CCR8 ligand (e.g., CCL1) against CCR8.

An anti-CCR8 antibody is preferably an antibody that strongly recognizes human CCR8. When selecting the antibody that strongly recognizes human CCR8, an antibody or an antibody fragment thereof that more strongly recognizes human CCR8 can be selected by selecting the antibody or the antibody fragment thereof using the strength of neutralizing activity as an index.

An amino acid sequence of an anti-CCR8-scFV region can be determined using amino acid sequence information on a CDR or a heavy/light chain variable region of an anti-CCR8 antibody. The light chain variable region and the heavy chain variable region in the anti-CCR8-scFv region may be linked via a linker sequence. An example of the linker sequence is the sequence of SEQ ID NO: 2. Examples of the amino acid sequence of the anti-CCR8-scFv region include the sequences of SEQ ID NOs: 39 to 44.

An anti-CCR8-scFv region preferably comprises any of the following sequences:

1) a light chain variable region including

CDR1 having the amino acid sequence of SEQ ID NO: 57,
CDR2 having the amino acid sequence of SEQ ID NO: 58, and
CDR3 having the amino acid sequence of SEQ ID NO: 59, and
a heavy chain variable region including
CDR1 having the amino acid sequence of SEQ ID NO: 60,
CDR2 having the amino acid sequence of SEQ ID NO: 61, and

CDR3 having the amino acid sequence of SEQ ID NO: 62;

2) a light chain variable region including

CDR1 having the amino acid sequence of SEQ ID NO: 57,
CDR2 having the amino acid sequence of SEQ ID NO: 63, and
CDR3 having the amino acid sequence of SEQ ID NO: 59, and
a heavy chain variable region including
CDR1 having the amino acid sequence of SEQ ID NO: 64,
CDR2 having the amino acid sequence of SEQ ID NO: 61, and
CDR3 having the amino acid sequence of SEQ ID NO: 65;

3) a light chain variable region including

CDR1 having the amino acid sequence of SEQ ID NO: 66,
CDR2 having the amino acid sequence of SEQ ID NO: 67, and
CDR3 having the amino acid sequence of SEQ ID NO: 68, and
a heavy chain variable region including
CDR1 having the amino acid sequence of SEQ ID NO: 69,
CDR2 having the amino acid sequence of SEQ ID NO: 70, and
CDR3 having the amino acid sequence of SEQ ID NO: 71;

4) a light chain variable region including

CDR1 having the amino acid sequence of SEQ ID NO: 72,
CDR2 having the amino acid sequence of SEQ ID NO: 67, and
CDR3 having the amino acid sequence of SEQ ID NO: 73, and
a heavy chain variable region including
CDR1 having the amino acid sequence of SEQ ID NO: 74,
CDR2 having the amino acid sequence of SEQ ID NO: 75, and
CDR3 having the amino acid sequence of SEQ ID NO: 76; or

5) a light chain variable region including

CDR1 having the amino acid sequence of SEQ ID NO: 77,
CDR2 having the amino acid sequence of SEQ ID NO: 78, and
CDR3 having the amino acid sequence of SEQ ID NO: 79, and
a heavy chain variable region including
CDR1 having the amino acid sequence of SEQ ID NO: 80,
CDR2 having the amino acid sequence of SEQ ID NO: 81, and
CDR3 having the amino acid sequence of SEQ ID NO: 82.

The anti-CCR8-scFv region more preferably comprises any of the following sequences:

1) a light chain variable region having the amino acid sequence of SEQ ID NO: 45, and
a heavy chain variable region having the amino acid sequence of SEQ ID NO: 46;
2) a light chain variable region having the amino acid sequence of SEQ ID NO: 47, and
a heavy chain variable region having the amino acid sequence of SEQ ID NO: 48;
3) a light chain variable region having the amino acid sequence of SEQ ID NO: 49, and
a heavy chain variable region having the amino acid sequence of SEQ ID NO: 50;
4) a light chain variable region having the amino acid sequence of SEQ ID NO: 51, and
a heavy chain variable region having the amino acid sequence of SEQ ID NO: 52;
5) a light chain variable region having the amino acid sequence of SEQ ID NO: 53, and
a heavy chain variable region having the amino acid sequence of SEQ ID NO: 54; or
6) a light chain variable region having the amino acid sequence of SEQ ID NO: 55, and
a heavy chain variable region having the amino acid sequence of SEQ ID NO: 56.

Examples of an effector cell of "a region comprising one or more of a light chain variable region or a heavy chain variable region of an antibody that recognizes a surface marker of an effector cell" include a T cell, an NK cell, and a macrophage, and a T cell or an NK cell is preferred. Examples of a surface marker of a T cell include immune checkpoint molecule such as CD3, OX40, 4-1BB, TIGIT, LAG3, GITR, ICOS, and CD28, a surface marker of a NK cell include CD16, TIGIT, 4-1BB, and LAG3, and a surface marker of a macrophage include CD64, CD40, and 4-1BB. A surface marker of the effector cell is preferred CD3 or CD16.

Examples of an amino acid sequence of a region comprising one or more of a light chain variable region or a heavy chain variable region of an antibody that recognizes a surface marker of an effector cell, when a surface marker is human CD3, include the sequence of SEQ ID NO: 7 described in Patent Document 27, the sequence of SEQ ID NO: 9 described in Patent Document 28, the sequence of SEQ ID NO: 8 described in Patent Document 29, when a surface marker is mouse CD3, include the sequence of SEQ ID NO: 84 described in paper by Fernandes et al (J. Biol. Chem., (2012), 287(16), 13324-13335), when a surface marker is mouse CD16, include the sequence of SEQ ID NO: 34 described in Patent Document 30.

An anti-CD3-scFV region is an scFV fragment derived from an antibody thar recognizes CD3 and preferably comprises any of the following sequences:

1) a light chain variable region including

CDR1 having the amino acid sequence of SEQ ID NO: 16,
CDR2 having the amino acid sequence of SEQ ID NO: 17, and
CDR3 having the amino acid sequence of SEQ ID NO: 18, and
a heavy chain variable region including
CDR1 having the amino acid sequence of SEQ ID NO: 19,
CDR2 having the amino acid sequence of SEQ ID NO: 20, and
CDR3 having the amino acid sequence of SEQ ID NO: 21;

2) a light chain variable region including

CDR1 having the amino acid sequence of SEQ ID NO: 22,
CDR2 having the amino acid sequence of SEQ ID NO: 23, and
CDR3 having the amino acid sequence of SEQ ID NO: 24, and
a heavy chain variable region including
CDR1 having the amino acid sequence of SEQ ID NO: 25,
CDR2 having the amino acid sequence of SEQ ID NO: 26, and
CDR3 having the amino acid sequence of SEQ ID NO: 27;

3) a light chain variable region including

CDR1 having the amino acid sequence of SEQ ID NO: 28,
CDR2 having the amino acid sequence of SEQ ID NO: 29, and
CDR3 having the amino acid sequence2 of SEQ ID NO: 30, and
a heavy chain variable region including
CDR1 having the amino acid sequence of SEQ ID NO: 31,
CDR2 having the amino acid sequence of SEQ ID NO: 32, and
CDR3 having the amino acid sequence of SEQ ID NO: 33; or

4) a light chain variable region including

CDR1 having the amino acid sequence of SEQ ID NO: 87,
CDR2 having the amino acid sequence of SEQ ID NO: 88, and
CDR3 having the amino acid sequence of SEQ ID NO: 89, and
a heavy chain variable region including
CDR1 having the amino acid sequence of SEQ ID NO: 90,
CDR2 having the amino acid sequence of SEQ ID NO: 91, and
CDR3 having the amino acid sequence of SEQ ID NO: 92.

The anti-CD3-scFv region more preferably comprises any of the following sequences:

1) a light chain variable region having the amino acid sequence of SEQ ID NO: 10, and

a heavy chain variable region having the amino acid sequence of SEQ ID NO: 11;
2) a light chain variable region having the amino acid sequence of SEQ ID NO: 12, and
a heavy chain variable region having the amino acid sequence of SEQ ID NO: 13;
3) a light chain variable region having the amino acid sequence of SEQ ID NO: 14, and
a heavy chain variable region having the amino acid sequence of SEQ ID NO: 15; or
4) a light chain variable region having the amino acid sequence of SEQ ID NO: 85, and
a heavy chain variable region having the amino acid sequence of SEQ ID NO: 86.

Also, an anti-CD3-VHH region is a VHH fragment derived from a VHH antibody that recognizes CD3.

An anti-CD16-scFV region is an scFv fragment derived from an antibody that recognizes CD16. Also, an anti-CD16-VHH region is a VHH fragment derived from a VHH antibody that recognizes CD16 and preferably comprises a heavy chain variable region including a CDR1 having the amino acid sequence of SEQ ID NO: 35, a CDR2 having the amino acid sequence of SEQ ID NO: 36, and a CDR3 having the amino acid sequence of SEQ ID NO: 37.

An anti-CD64-scFV region is an scFv fragment derived from an antibody that recognizes CD64. Also, an anti-CD64-VHH region is a VHH fragment derived from a VHH antibody that recognizes CD64.

The bispecific antibody of the present invention may comprise a signal sequence region at an N-terminal. The signal sequence is preferred a CD8A signal sequence, and the CD8A signal sequence region may be present on an N-terminal side of an anti-CCR8-scFv region. Furthermore, the CD8A signal sequence region preferably has the amino acid sequence of SEQ ID NO: 1.

"An anti-CCR8-scFv region comprising a light chain variable region and a heavy chain variable region of an anti-CCR8 antibody" and "a region comprising one or more of a light chain variable region or a heavy chain variable region of an antibody that recognizes a surface marker of an effector cell" may be linked via an scFv linker sequence. Examples of the scFv linker sequence include the sequences of SEQ ID NOs: 3 to 6.

The bispecific antibody of the present invention may be linked to a tag at an N-terminal or a C- terminal and when the bispecific antibody is linked to a tag, the bispecific antibody is preferably inked to a tag at C-terminal. Examples of the tag be linked include histidine, FLAG, Glutathione-S-transferase, and hemagglutinin, and the histidine tag is preferred. Furthermore, the histidine tag has the amino acid sequence of SEQ ID NO: 38.

As the bispecific antibody of the present invention, a CD8A signal sequence region, an anti-CCR8-scFv region, an scFv linker, a region comprising one or more of a light chain variable region or a heavy chain variable region of an antibody that recognizes a surface marker of an effector cell, and a histidine tag were preferably linked in this order from an N-terminal side.

The bispecific antibody in the present invention may have an Fc region. The Fc region is preferably a single chain, preferably has low antibody dependent cellular cytotoxicity (ADCC) activity such as Fc regions of a human IgG2 antibody or a human IgG4 antibody, and is preferably inserted just before a histidine tag. An Example of the Fc region includes the Fc region having the amino acid sequence of SEQ ID NO: 95.

Examples of the bispecific antibody of the present invention include a bispecific antibody that recognizes CCR8 and CD3 as an antigen (CCR8-CD3 bispecific antibody), a bispecific antibody that recognizes CCR8 and CD16 as an antigen (CCR8-CD16 bispecific antibody), and a bispecific antibody that recognizes CCR8 and CD64 as an antigen (CCR8-CD64 bispecific antibody), but these are not limited.

Each region comprised in the bispecific antibody of the present invention can exhibit a similar effect as long as it has 90% or more identity, preferably 95% or more identity in an amino acid sequence.

Effector cells exhibit strong cytotoxic activity against target cells, by the bispecific antibody binding a target cell to an effector cell. The "cytotoxic activity" includes cell phagocytic activity by macrophages and the like.

Examples of CCR8-expressing cells in which the bispecific antibody of the present invention includes cytotoxic activity include Treg cells such as tumor-infiltrating Treg cells and tumor cells.

When a CCR8-expressing cell is a tumor-infiltrating Treg cell, the tumor is preferably a solid cancer. Specific examples of the solid cancer include breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, bile duct carcinoma, gallbladder carcinoma, thyroid carcinoma, testicular cancer, thymic carcinoma, hepatocarcinoma, skin cancer, and brain tumor. Preferably, examples thereof include breast cancer, uterine corpus cancer, ovarian cancer, lung cancer, bladder cancer, colorectal cancer, kidney cancer, sarcoma, hepatocarcinoma, skin cancer, and brain tumor. When the CCR8-expressing cell is the tumor-infiltrating Treg cell, no CCR8 may be expressed in the tumor cell.

When a CCR8-expressing cell is a tumor cell, the tumor is preferably melanoma or hematological cancer. Examples of the hematological cancer expressing CCR8 include leukemias such as acute myeloid leukemia (AML) and acute lymphocytic leukemia (ALL), and lymphomas such as adult T-cell leukemia lymphoma (ATLL), peripheral T-cell lymphoma (PTCL), cutaneous T-cell lymphoma (CTCL), diffuse large B-cell lymphoma (DLBCL), and Hodgkin lymphoma.

The bispecific antibody of the present invention is useful as a pharmaceutical composition. A pharmaceutical composition of the present invention can be administered systemically or topically, orally or parenterally. Examples of the parenteral administration include intravenous injection, such as infusion, intramuscular injection, intraperitoneal injection, subcutaneous injection, intranasal administration, and inhalation.

The pharmaceutical composition of the present invention is very useful as a medicament for the treatment and/or prevention of a CCR8-related disease. In particular, the pharmaceutical composition is very useful as a medicament for treating and/or preventing cancer in which intratumoral infiltration of CCR8-expressing Treg cells has occurred or tumor cells express CCR8. For example, the pharmaceutical composition of the present invention is very useful as a medicament for treating and/or preventing cancer such as breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, bile duct carcinoma, gallbladder carcinoma, thyroid carcinoma, testicular cancer, thymic carcinoma, hepatocarcinoma, skin cancer, and brain tumor, preferably breast cancer, uterine corpus cancer, ovary cancer, lung cancer, bladder cancer, colorectal cancer, kidney cancer, sarcoma, hepatocarcinoma, skin cancer, and brain tumor. The pharmaceutical composition is also very useful as a medicament for treating and/or preventing melanoma, leukemia and lymphoma.

The cancer according to the present invention includes all solid cancers and hematological cancers. Specific examples of the cancer include breast cancer, uterine corpus cancer, cervical cancer, ovarian cancer, prostate cancer, lung cancer, stomach cancer (gastric adenocarcinoma), non-small cell lung cancer, pancreatic cancer, head and neck squamous cell cancer, esophageal cancer, bladder cancer, melanoma, colorectal cancer, kidney cancer, non-Hodgkin lymphoma, urothelial cancer, sarcoma, blood cell carcinoma (leukemia, lymphoma, etc.), bile duct carcinoma, gallbladder carcinoma, thyroid carcinoma, testicular cancer, thymic carcinoma, hepatocarcinoma, skin cancer, and brain tumor. Preferably, examples thereof include breast cancer, uterine corpus cancer, ovarian cancer, lung cancer, bladder cancer, colorectal cancer, kidney cancer, sarcoma, hepatocarcinoma, skin cancer, and brain tumor. Melanoma, leukemia, and lymphoma are also preferred.

Note that the cancer according to the present invention shall mean not only epithelial malignancies such as ovarian cancer and stomach cancer, but also non-epithelial malignancies including hematopoietic cancer such as chronic lymphocytic leukemia and Hodgkin lymphoma. Furthermore, in the present description, the terms such as "cancer," "carcinoma," "tumor," and "neoplasm" are not distinguishable from one another and used interchangeably.

The bispecific antibody of the present invention may be used in combination with other agents and administered as a concomitant agent, for

(1) supplementing and/or enhancing the therapeutic effect of the pharmaceutical composition of the present invention,
(2) improving in kinetics or absorption or reducing in dosage of the pharmaceutical composition of the present invention, and/or
(3) reducing the side effects of the pharmaceutical composition of the present invention.

The concomitant agent of the bispecific antibody of the present invention in combination with other agents may be administered in the form of a combination drug containing both components in one formulation, or may be administered in separate formulations. When administered in separate formulations, they can be co-administered or administered with time difference. The administration with time difference may be performed by administering the bispecific antibody of the present invention, then the other agent, or by administering the other agent, then the bispecific antibody of the present invention, in which each administration method may be the same or different.

It is contemplated that a patient to be subjected to the pharmaceutical composition of the present invention is or is suspected of being a cancer patient. The effective dosage of the pharmaceutical composition according to the present invention is selected from the range of 0.01 mg to 100 mg per kg of body weight per dose. Alternatively, it can be selected from a dosage of 5 to 5000 mg, preferably 10 to 500 mg, per patient. However, the dosage of the pharmaceutical composition comprising the bispecific antibody of the present invention are not limited to these dosages. The administration period can also be appropriately selected depending on the age or symptoms of the patient. The pharmaceutical composition of the present invention may further comprise a pharmaceutically acceptable carrier or additive, depending on the route of administration. Examples of the carrier and additive include water, a pharmaceutically acceptable organic solvent, collagen, polyvinyl alcohol, polyvinylpyrrolidone, sodium alginate, water-soluble dextran, pectin, methylcellulose, ethylcellulose, casein, diglycerin, propylene glycol, polyethylene glycol, petrolatum, human serum albumin (HSA), mannitol, sorbitol, lactose, and a surfactant acceptable as a pharmaceutical additive. The additives used are selected as appropriate or in combination from the above, depending on the dosage form, but are not limited thereto.

The present invention encompasses a polynucleotide encoding an amino acid sequence of the present invention. The present invention further encompasses an expression vector comprising the polynucleotide.

The polynucleotide is not particularly limited as long as it encodes the bispecific antibody of the present invention, and it is a polymer consisting of nucleotides such as a plurality of deoxyribonucleic acids (DNA) or ribonucleic acids (RNA). It may contain a non-natural nucleotide. The polynucleotide of the present invention can be used for producing antibodies by genetically engineered methods.

[EXAMPLES]

Hereinafter, the present invention will be explained in more detail by way of Examples of the present invention, but the present invention is not limited by them.

A CDR sequence of each antibody was identified by conducting Kabat numbering about amino acid sequences of a light chain and a heavy chain of an antibody using an antibody sequence analysis software abYsis.

Antibiotics such as penicillin, streptomycin, and kanamycin were appropriately added to each cell culture medium.

Example 1: Production of anti-human CCR8 antibody and anti-CCR8-scFv region

An anti-human CCR8 antibody was obtained based on the methods described in Examples 1 to 2, 4 to 7, and 9 of WO 2020/138489. Furthermore, an anti-CCR8-scFv region was produced linking a heavy chain variable region of the anti-human CCR8 antibody, the G4S peptide linker (SEQ ID NO: 2), and a light chain variable region in this order. Table 1 shows SEQ ID NOs of a light chain variable region, a heavy chain variable region, and a CDR of the anti-human CCR8 antibody, and the SEQ ID NOs of the anti-CCR8-scFv region prepared from each antibody.

Here, αCCR8-B1 is a humanized monoclonal antibody, αCCR8-B2 to 4 are mouse-derived monoclonal antibodies, and αCCR8-B5 and αCCR8-B6 are rat-derived monoclonal antibodies. αCCR8-B1, αCCR8-B4, and αCCR8-B6 are the antibodies described in Examples of WO 2020/138489, αCCR8-B2, and αCCR8-B3 are antibodies described in Examples of WO 2022/211046. The amino acid sequences of the light chain variable region, the heavy chain variable region, and the CDRs of each antibody are also listed in the sequence listings of the corresponding patent documents mentioned above and are publicly known.

[Table 1]

| mAb | | SEQ ID NO: | | SEQ ID NO: | | SEQ ID NO: | | SEQ ID NO: |
|---|---|---|---|---|---|---|---|---|
| αCCR8-B1 scFv: SEQ ID No.39 | Light chain variable region | 45 | CDR1 | 57 | Heavy chain variable region | 46 | CDR1 | 60 |
| | | | CDR2 | 58 | | | CDR2 | 61 |
| | | | CDR3 | 59 | | | CDR3 | 62 |
| αCCR8-B2 scFv: SEQ ID No.40 | Light chain variable region | 47 | CDR1 | 57 | Heavy chain variable region | 48 | CDR1 | 64 |
| | | | CDR2 | 63 | | | CDR2 | 61 |
| | | | CDR3 | 59 | | | CDR3 | 65 |
| αCCR8-B3 scFv: SEQ ID No.41 | Light chain variable region | 49 | CDR1 | 57 | Heavy chain variable region | 50 | CDR1 | 64 |
| | | | CDR2 | 63 | | | CDR2 | 61 |
| | | | CDR3 | 59 | | | CDR3 | 65 |
| αCCR8-B4 scFv: SEQ ID No.42 | Light chain variable region | 51 | CDR1 | 66 | Heavy chain variable region | 52 | CDR1 | 69 |
| | | | CDR2 | 67 | | | CDR2 | 70 |
| | | | CDR3 | 68 | | | CDR3 | 71 |
| αCCR8-B5 scFv: SEQ ID No.43 | Light chain variable region | 53 | CDR1 | 72 | Heavy chain variable region | 54 | CDR1 | 74 |
| | | | CDR2 | 67 | | | CDR2 | 75 |
| | | | CDR3 | 73 | | | CDR3 | 76 |
| αCCR8-B6 scFv: SEQ ID No.44 | Light chain variable region | 55 | CDR1 | 77 | Heavy chain variable region | 56 | CDR1 | 80 |
| | | | CDR2 | 78 | | | CDR2 | 81 |
| | | | CDR3 | 79 | | | CDR3 | 82 |

Example 2: Production of bispecific antibody comprising anti-CCR8-scFv region and anti-CD3-scFv region

(1) Anti-CD3-scFv region

The anti-CD3-scFv region comprising a light chain variable region and a heavy chain variable region in an anti-human CD3 antibody in table 2 or an anti-mouse CD3 antibody in table 3 was used for production of the bispecific antibody.

Amino acid sequences of the light chain variable region, the heavy chain variable region, and the anti-CD3-scFv region in the anti-human CD3 antibody in table 2 are described in WO99/54440 for αCD3-1, in WO2017/134158 for αCD3-2, and in WO2016/182751 for αCD3-3. The anti-mouse CD3 antibody in table 3 is described in the document of Fernandes et al. (J. Biol. Chem., (2012), 287(16), 13324-13335) and the anti-CD3-scFv region was produced linking the heavy chain variable region, the G4S peptide linker (SEQ ID NO: 2) and the light variable region in this order.

[Table 2]

| mAb | | SEQ ID NO: | | SEQ ID NO: | | SEQ ID NO: | | SEQ ID NO: |
|---|---|---|---|---|---|---|---|---|
| αCD3-1 scFv: SEQ ID No.7 | Light chain variable region | 10 | CDR1 | 16 | Heavy chain variable region | 11 | CDR1 | 19 |
| | | | CDR2 | 17 | | | CDR2 | 20 |
| | | | CDR3 | 18 | | | CDR3 | 21 |
| αCD3-2 scFv: SEQ ID No.8 | Light chain variable region | 12 | CDR1 | 22 | Heavy chain variable region | 13 | CDR1 | 25 |
| | | | CDR2 | 23 | | | CDR2 | 26 |
| | | | CDR3 | 24 | | | CDR3 | 27 |
| αCD3-3 scFv: SEQ ID No.9 | Light chain variable region | 14 | CDR1 | 28 | Heavy chain variable region | 15 | CDR1 | 31 |
| | | | CDR2 | 29 | | | CDR2 | 32 |
| | | | CDR3 | 30 | | | CDR3 | 33 |

[Table 3]

| mAb | | SEQ ID NO: | | SEQ ID NO: | | SEQ ID NO: | | SEQ ID NO: |
|---|---|---|---|---|---|---|---|---|
| αmCD3 scFv SEQ ID No.84 | Light chain variable region | 85 | CDR1 | 87 | Heavy chain variable region | 86 | CDR1 | 90 |
| | | | CDR2 | 88 | | | CDR2 | 91 |
| | | | CDR3 | 89 | | | CDR3 | 92 |

(2) Production of bispecific antibody

The bispecific antibody (CCR8-CD3 bispecific antibody) comprising the anti-CCR8-scFv region and the anti-CD3-scFv region was produced linking the CD8A signal sequence region (SEQ ID NO: 1), the anti-CCR8-scFv region of example 1, the scFv linker (SEQ ID NO: 3), the anti-CD3-scFv region of (1), and the histidine tag (SEQ ID NO: 38) in this order from an N-terminal side. When the anti-CD3-scFv region is the sequence of SEQ ID NO: 8, 9, or 84, the sequence of SEQ ID NO: 4 instead of SEQ ID NO: 3 was used as the scFv linker.

A Polynucleotide encoding each bispecific antibody was introduced into a mammalian expression vector (a vector obtained by converting a CMV promoter of a pQCXIP vector of Takara Bio Inc. into a human EF promoter), 293T cells were transformed using lipofectamine 3000. From 3 days after transduction, drug selection was performed with puromycin (1 μg/ml) for a week. After expansion culture of the drug-resistant cells were carried out and 293T cells that reached confluence were cultured for an additional two days, 50 ml of the cell culture medium was recovered. The recovered medium was passed through a 45 μm filter and then concentrated using Amicon Ultra-4 filter (10 kDa cut-off) of milipore. To completely remove the puromycin, after concentration, the operations of 10-fold dilution with PBS and subsequent centrifugal concentration were further repeated three times. Finally, the medium was concentrated at 25 to 50 times. An amount of protein of the bispecific antibody contained in the concentrated cell culture medium was quantified using His tag ELISA detection kit (GenScript).

(3) Purification of bispecific antibody

The bispecific antibodies generated in Examples 20 to 23 were purified by the following methods.

Recovering the cell culture liquid after drug-selection obtained in (2), the cell culture liquid was concentrated at 35 times by ultrafiltration with the Amicon Ultra-15 (Millipore) at a cutoff of 30 kDa. After concentration, using HIS tag resin (TALON, Takara), the protein was purified according to manufacturer's protocol. Thereafter, buffer was replaced to PBS by using Amicon Ultra-15 and quantified. The protein was quantified according to manufacturer's protocol using HIS-TAG quantitative kit (His tag ELISA detection kit, GenScript). Analyzing the bispecific antibody by western blotting, a specific band was detected at the desired size (55 kDa), it was confirmed that the protein could be generated without being decomposed.

Example 3: Production of bispecific antibody comprising anti-CCR8-scFv region and anti-CD16-VHH region

(1) anti-CD16-VHH region

The anti-CD16-VHH region described in WO2020/081841 was used for production of the bispecific antibody. The anti-CD16-VHH region has the sequence of SEQ ID NO: 34, CDR1, CDR2, and CDR3 have the sequence pf SEQ ID NO: 35, 36, and 37 respectably.

(2) Production of bispecific antibody

The bispecific antibody (CCR8-CD16 bispecific antibody) comprising the anti-CCR8-scFv region and the anti-CD16-VHH region was produced replacing an anti-CD3-scFv region to the anti-CD16-VHH region in the same manner as in Example 2 (2). The sequence of SEQ ID NO: 4 was used as an scFv linker.

Example 4: Cytotoxic activity of CCR8-CD3 bispecific antibody against human CCR8-expressing cells using PBMC as effector cells.

(1) Preparation of effector cells

Peripheral blood mononuclear cells (PBMCs) were stimulated for three days using CD3/CD28 beads (Dynabeads Human T-activator CD3/28,GIBCO) and human IL-2 (Peprotech, final concentration 100 U/ml), and stocked in an ultra-low temperature freezer. The cells were thawed, stimulated in the same conditions for five days, and grown. Thereafter, the cells were divided into 2 groups. One group was further cultured for 24 hours removing the beads and adding only IL-2 at 100 U/ml. The other group was further cultured for 24 hours in the presence of IL-2 and a stimulated state without removing the beads. These cells were washed twice with an assay buffer (4 % FCS/DMEM (phenol red-free)) and used for evaluation of cytotoxic activity.

(2) Preparation of target cells

Rat-1 cells that are forcibly expressing a human CCR8 gene by a CMV promoter and their mother cells, Rat-1 cells, were used. These cells were cultured in 10 % FCS/DMEM culture medium. Both cells were labeled adding calcein at a concentration of $1\times10^6$/ml. The cells were cultured at 37 °C for 30 minutes and washed twice with the assay buffer. The cells were used for evaluation of cytotoxic activity.

(3) Evaluation of cytotoxic activity

Evaluation of cytotoxic activity was performed using the bispecific antibody (B1-CD3-1) comprising an anti-CCR8-scFv region derived from $\alpha$CCR8-B1 and an anti-CD3-scFv region derived from $\alpha$CD3-1 as a CCR8-CD3 bispecific antibody.

The Effector cells were mixed so as to be $5\times10^4$/150 $\mu$L/well, the target cells were mixed so as to be $5\times10^3$/well/150 $\mu$L, the CCR8-CD3 bispecific antibody was mixed so as to have a final concentration of 1.7 nM or 0 nM in a 96 well plate. After five hours, the cell culture solution of 35 $\mu$L was separated and an amount of calcein in medium was quantified by a fluorometer. Assuming that a spontaneous release amount of calcein after five hours in the case of each target cell only was 0 % and a total calcein amount after five hours was 100 %, killing rate (%) was calculated (N=1).

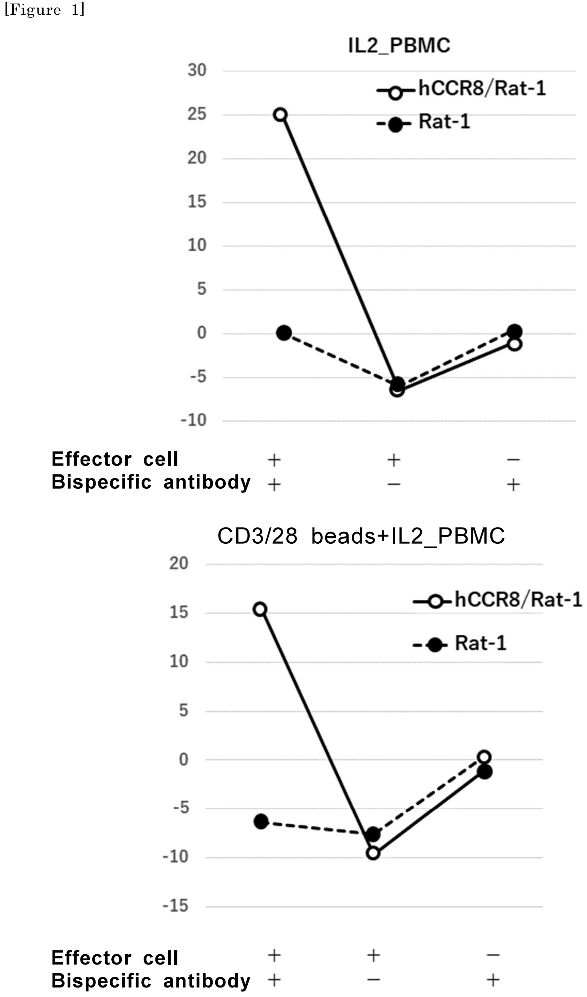

The results are shown in Figure 1. Only when both the effector cells and the CCR8-CD3 bispecific antibody were added, killing of hCCR8/Rat-1 cells was observed. From the above, it was found that the CCR8-CD3 bispecific antibody has cytotoxic activity against target cells dependently on effector cells and CCR8 expression.

Example 5: Cytotoxic activity of CCR8-CD3 bispecific antibody against human CCR8-expressing cells using unstimulated PBMCs as effector cells.

(1) Preparation of effector cells

Human PBMCs (Precision) was cultured for three days using human IL-2 (Peprotech, final concentration 100 U/ml) and the cell state was made stable.

(2) Preparation of target cells

A human CCR8 gene and a firefly luciferase gene expressing Rat-1 cells (hCCR8/FLuc/Rat-1 cells) by a CMV promoter were used.

(3) Evaluation of cytotoxic activity

Evaluation of cytotoxic activity was performed using B1-CD3-1 and B5-CD3-1 (a bispecific antibody comprising an anti-CCR8-scFv region derived from αCCR8-B5 and an anti-CD3-scFv region derived from αCD3-1) as the CCR8-CD3 bispecific antibody. Blinatumomab was used as a negative control.

The effector cells were used at $1\times10^4$/well/60 $\mu$L, the target cells were used at $1\times10^3$/well/60 $\mu$L, so that a ratio of the effector cells to the target cells (E/T cell ratio) was 10:1. The CCR8-CD3 bispecific antibody was used at a final concentration of 1.6 nM, the blinatumomab was used at a final concentration of 0.3 nM. The effector cells and target cells suspended in an assay buffer were mixed with the CCR8-CD3 bispecific antibody and cultured in a 96 well plate for 24 hours (N=1), 48 hours (N=2). Thereafter, 140 $\mu$L of the assay buffer was added, and after centrifugation, 100 $\mu$L of the supernatant removed to replace cell culture medium. 50 $\mu$L of firefly luciferase substrate (Bio-Glo Luciferase Assay, promega) was added to the cells, the cells were transferred to a white light proof plate and luciferase activity was measured after 15 minutes. By replacing the cell culture medium, it is possible to remove luciferase protein which was released into the medium because of cell death and only luciferase activity in the living cells was evaluated quantitatively.

The effector cells and the target cells were mixed and killing rate (%) was calculated, assuming that a luciferase value without the CCR8-CD3 bispecific antibody was 0% of a killing rate by the CCR8-CD3 bispecific antibody and a luciferase value was 100% of a killing rate when the cells completely killed, and killing rate (%) was calculated.

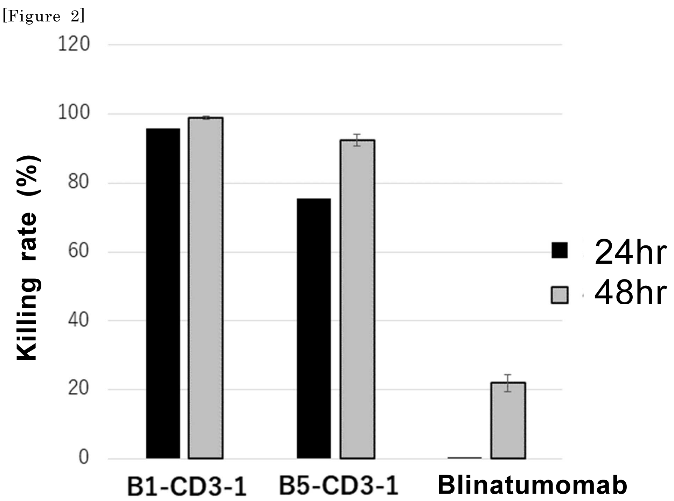

The results are shown in Figure 2. When the CCR8-CD3 bispecific antibody was not added and the blinatumomab was added, cytotoxic activity was hardly observed in both cases of 24 and 48 hours. On the other hand, the B1-CD3-1 and B5-CD3-1 showed nearly 70~100% of cytotoxic activity in the cases of both times. From the above, it was found that the CCR8-CD3 bispecific antibody shows cytotoxic activity against the target cells dependently on the anti-CCR8-scFv region even when non-stimulated PBMCs were used.

Example 6: Cytotoxic activity of CCR8-CD3 bispecific antibody against human CCR8 expressing cells using CD8-positive T cells as effector cells

(1) Preparation of effector cells

CD8-positive T cells were separated from frozen human PBMCs (ACCUCELL) according to the protocol using MojoSort™ Human CD8 T Cell Isolation Kit (BioLegend). These cells were stimulated for 8 days using Dynabeads Human T-Activator CD3/CD28 (GIBCO) and human IL-2 (100 U/ ml). Beads: cells ratio was 1: 5. The stimulated cells were cryopreserved as they were and used in each subsequent experiment. The cells were thawed, the beads were removed with a magnet and the cells cultured for 5 days at IL-2 (100 U/ml).

(2) Evaluation of cytotoxic activity

The same hCCR8/FLuc/Rat-1 cells as the target cells in Example 5 were used as target cells. Evaluation of cytotoxic activity was performed using the bispecific antibody in Table 4 as the CCR8-CD3 bispecific antibody.

The effector cells were used at $3\times10^4$/well/60 $\mu$L, the target cells were used at $3\times10^3$/well/60 $\mu$L, so that a ratio of the effector cells to the target cells (E/T cell ratio) was 10:1. A concentration of the CCR8-CD3 bispecific antibody was evaluated by 3 times dilution series from 15.9 pM (N=2). Each cell and the CCR8-CD3 bispecific antibody were mixed and cultured at 37 °C for 6 hours, the cells were washed in the same manner as in Example 5, and luciferase activity of the cells was similarly measured. Killing rate was also calculated in the same manner as in Example 5.

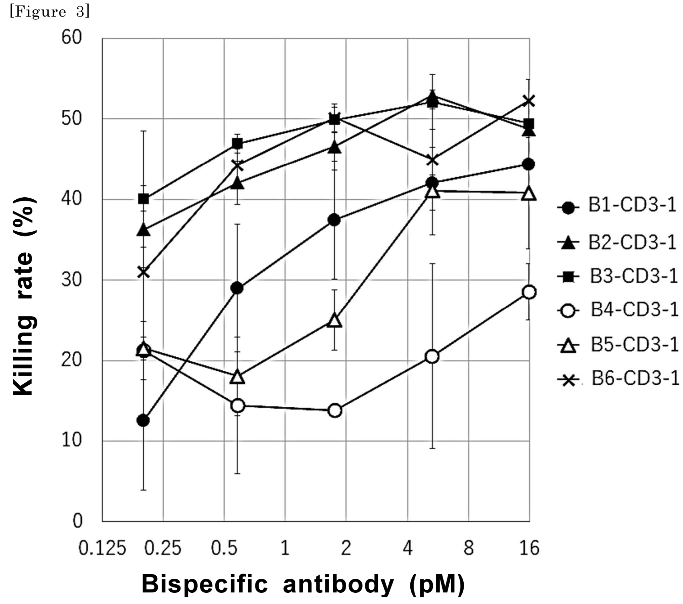

The results are shown in Figure 3. Furthermore, EC 50 vale was calculated from average value of two measured values at each concentration. The analysis was performed using R software's drc package (Analysis of Dose-Response Curves). EC50 was estimated by Three-variable log-logistic analysis. Maximum of killing rate was estimated by the same package. As a result, according to Table 5, EC50 values of B2-CD3-1, B3-CD3-1, and B6-CD3-1 were the same extent, B1-CD3-1 and B5-CD3-1 were higher than these, and B4-CD3-1 was the highest. The maximum of the killing rate for assay time of 6 hours showed a similar trend to EC50 value.

[Table 4]

| Bispecific antibody | Anti-CCR8-scFv | Anti-CD3-scFv |
|---|---|---|
| B1-CD3-1 | αCCR8-B1 | αCD3-1 |
| B2-CD3-1 | αCCR8-B2 | αCD3-1 |
| B3-CD3-1 | αCCR8-B3 | αCD3-1 |
| B4-CD3-1 | αCCR8-B4 | αCD3-1 |
| B5-CD3-1 | αCCR8-B5 | αCD3-1 |
| B6-CD3-1 | αCCR8-B6 | αCD3-1 |

[Table 5]

| Bispecific antibody | EC50 (pM) | Maximum of killing rate |
|---|---|---|
| B1-CD3-1 | 411.9 | 41.2 |
| B2-CD3-1 | 167.8 | 47.6 |
| B3-CD3-1 | 162.2 | 49.6 |
| B4-CD3-1 | 1951.6 | 28.4 |
| B5-CD3-1 | 1112.6 | 40.9 |
| B6-CD3-1 | 181.6 | 47.9 |

Example 7: Cytotoxic activity of CCR8-CD3 bispecific antibody against ATLL cell line

(1) Analysis of Foxp3 and CCR8 expression in ATLL cell line, ATN-1 cells

Adult T-cell leukemia lymphoma (ATLL) cell line, ATN-1 cells, were seeded on a 96-well plate at $1 \times 10^5$ cells/well. An HBSS/2% FCS/10 mM HEPES buffer containing an amount of 1/20 of Human TruStain FcX (BioLegend) and an amount of 1/500 of LIVE/DEAD (Thermo Fisher) was added to each well, and allowed to stand at room temperature for 15 minutes. Thereafter, the cells were washed once with an HBSS/2% FCS/10 mM HEPES buffer, suspended in 100 μl of the same buffer, and then stained at 4°C for 30 minutes using Alexa Fluor 700 -labeled anti-human CD3 (BioLegend), PE-labeled anti-human CCR8 antibody (BioLegend), or each fluorescently labeled isotype control antibody (BioLegend). The cells were washed twice with a HBSS/2% FCS/10 mM HEPES buffer, and then cell fixation was performed using Foxp3/Transcription Factor Staining Buffer Set (eBioscience). The cells were stained at 4°C for 30 minutes using APC-labeled FOXP3 antibody (eBioscience) or APC-labeled isotype control antibody (BioLegend). The cells were washed twice, expressions of each molecule were measured by a flow cytometer.

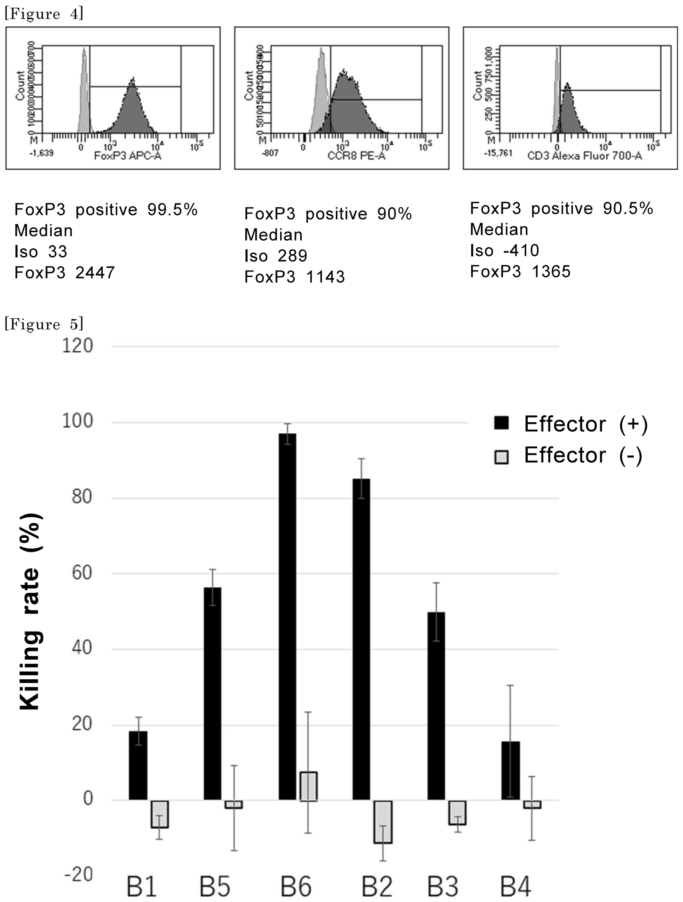

The results are shown in Figure 4. The ATN-1 cells are CD4-positive T cells and express large amounts of Foxp3, so the ATN-1 cells were considered to be human-Treg cell-derived cancer cell lines. Furthermore, it was confirmed that CCR8 was expressed in approximately 90% of the ATN-1 cells.

(2) Evaluation of cytotoxic activity

The ATN-1 cells express both CCR8 and CD3 molecule on the same cell, like human intratumoral Treg cells, so it was considered to be possible that the CCR8-CD3 bispecific antibody binds to the CCR8 and CD3 molecule on the same cell and activates ATN-1 cells through signals from the CD3 molecule, and therefor there were concerns about activation of a CMV promoter and so on by activation of the cells and an effect on cell proliferation. To dispel the concerns, the following experiments were performed.

FLuc/ATN-1 cells into which a firefly luciferase gene expressing in the ATN-1 cells by the CMV promoter introduced stably were used as target cells. It was confirmed that luciferase activity is correlated with number of the cells, and the cells were used at $5 \times 10^3$/well/60 μL. CD8-positive T cells cultured in the same manner as in Example 6 were used

at $5 \times 10^4$ cells/well/60 μL as effector cells (E/T cell ratio =10: 1) (N=3). The six CCR8-CD3 bispecific antibodies in Table 4 were evaluated. All concentrations were set to 175 pM and killing rate after 24 hours in the cases where effector cells were added and where they were not added was calculated in the same manner as in Example 5. In cases with effector cells and without them, the killing rate each was calculated, assuming that kill rate was calculated as 0 % when the bispecific antibody was not added, and killing rate was calculated as 100 % when luciferase activity was 0.

The results are shown in Figure 5. In the case that the effector cells were not added, the effect of each CCR8-CD3 bispecific antibody were within a range of approximately ±10 %, compared to the case that the CCR8-CD3 bispecific antibody was not added. From the results, it was concluded that the bispecific antibody only did not show cytotoxic activity against the target cells, or was not activated, and increase in the luciferase gene expression that would activate the CMV promoter was also not observed. In the case that the effector cells were added, decrease in number of the target cells was observed only when the bispecific antibody was added, and cytotoxic activity of B2-CD3-1 and B6-CD3-1 is especially high.

Next, a concentration of the CCR8-CD3 bispecific antibody was sequentially diluted 3 times from 530 pM, and killing rate was evaluated in the same manner.

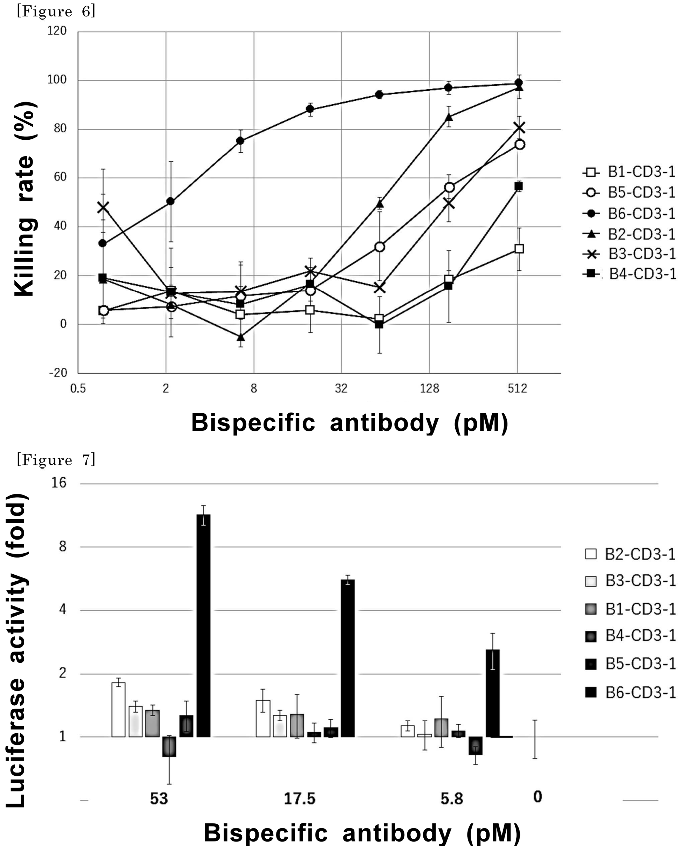

The results are shown in Figure 6. It was found that B6-CD3-1 has the strongest cytotoxic activity (N=2). EC50 value of each CCR8-CD3 bispecific antibody (Four-variable log-logistic analysis) were estimated by R software's drc package (Analysis of Dose-Response Curves), using average value of killing rate values at each concentration. Also, 95 % confidence interval at EC50 value was estimated by tfls method of the same package. Maximum of killing rate 24 hours after the assay were estimated by the same package (Table 6).

Regarding the EC50 values, B6-CD3-1 had the most significantly low EC50 value, 1.88 pM. The other bispecific antibodies had EC50 values 30 times or more than B6-CD3-1. Also, B6-CD3-1 and B2-CD3-1 are comparable in maximum of killing rate at 24 hours, and these are the highest of the six types. From the above, it was found that B6-CD3-1 has the strongest cytotoxic activity of the six types CCR8-CD3 bispecific antibody that were evaluated this time.

From this experiment, B6-CD3-1 exhibits the same level of cytotoxic activity as the other bispecific antibodies against the target cells that do not express the CD3 molecule, such as Rat-1 cells, but exhibits stronger cytotoxic activity than the other bispecific antibodies against target cells such as ATN-1 cells expressing the CD3 molecule. This result strongly suggests that B6-CD3-1 is more likely to form trans bonds (binding of CCR8 and CD3 molecules which are present on a cell membrane of the same cell) then cis bonds (binding of CCR8 and CD3 molecules between different cells) compared with the other bispecific antibodies. In other words, this indicates that a CCR8 binding mode of B6-CD3-1 is more likely to result in binding to CD3 present in trans, and therefore CCR8-expressing Treg cells are more likely to be damaged by effector cells. It would be difficult for a person skilled in the art to predict that a combination of anti-CCR8 and anti-CD3 antibody sequences is such a trans-dominant combination.

[Table 6]

| Bispecific antibody | EC50 (pM) | Standard error | 95% confidence interval lower limit (pM) | 95% confidence Interval Upper Limit (pM) | Maximum of killing rate |
|---|---|---|---|---|---|
| B1-CD3-1 | 174.1 | 19.2 | 128.2 | 236.4 | 30.9 |
| B2-CD3-1 | 61.8 | 17 | 28.8 | 132.4 | 98 |
| B3-CD3-1 | 174.9 | 27.1 | 113.7 | 268.9 | 80.9 |
| B4-CD3-1 | 230.3 | 455.9 | N.D. | N.D. | 56.7 |
| B5-CD3-1 | 131.6 | 69.3 | 30.5 | 567.3 | 94.7 |
| B6-CD3-1 | 1.88 | 0.16 | 1.5 | 2.37 | 99.8 |

(3) Evaluation of activation of target cells by signaling from CD3 molecule

It was evaluated whether it was confirmed that each CCR8-CD3 bispecific antibody was able to activate the cells through signaling from the CD3 molecule on T cells, and if so, which bispecific antibody had the most excellent activation potency.

Human T cell line, Jurkat (Jurkat-Luci NAFT Cells Kit, InvivoGen) cells, wherein a Lucia luciferase gene was linked at a downstream of an NFAT promoter and the degree of activation of the NFAT promoter colud be evaluated by the activity of the Lucia luciferase gene were used as effector cells. The same FLuc/ATN-1 cells as (2) were used as target cells. The effector cells and the target cells (both cells were at $2 \times 10^4$/well/100 μL) were mixed at 1:1 in a 96-well plate, and the CCR8-CD3 bispecific antibody was added by 3 times dilution series from a final concentration 53 pM (N=3). After 16 hours, the activity of Lucia luciferase was quantified according to the protocol of the above kit.

The results are shown in Figure 7. Assuming that the bispecific antibody non-added activity was 1, an activity of B6-CD3-1 increased by 11.4 times at 53 pM, 5.6 times at 17.5 pM, and 2.6 times at 5.8 pM. Activity of the other bispecific antibodies was under 2 times at any concentration and NFAT activation potency of B6-CD3-1 was higher than the other bispecific antibodies even when a concentration of B6-CD3-1 was about 10 times lower that the other bispecific antibody. Also, B2-CD3-1 had the next highest activity after B6-CD3-1 and cytotoxic activity and NFAT activation potency of each bispecific antibody were almost positively correlated. A significant difference test (T-test) was performed between B6-CD3-1, the other bispecific antibodies and no bispecific antibody at a concentration of each bispecific antibody. B6-CD3-1 showed significant differences against the other bispecific antibodies and no bispecific antibody in all concentration ranges (significance level:0.01 at 53 pM,0.001 at 17.5 pM and 0.05 at 5.8 pM).

From the above, it was found that B6-CD3-1 was the most excellent in both cytotoxic activity and NFAT activation potency from CD3.

Example 8: Amino acid sequence optimization of anti-CD3-scFv region in CCR8-CD3 bispecific antibody

B6-CD3-2 and B6-CD3-3 in Table 7 were produced by replacing an anti-CD3-scFv region of B6-CD3-1.

[Table 7]

| Bispecific antibody | Anti-CCR8-scFv | Anti-CD3-scFv |
|---|---|---|
| B6-CD3-1 | αCCR8-B6 | αCD3-1 |
| B6-CD3-2 | αCCR8-B6 | αCD3-2 |
| B6-CD3-3 | αCCR8-B6 | αCD3-3 |

hCCR8/FLuc/Rat-1 cells ($5\times10^3$/well/$\mu$L) used in Exmple 5 as tareget cells and PBMC cells activated in the same manner as in Example 4 as effector cells, were mixed in a 96-well plate at E/T cell ratio 3:1 and each CCR8-CD3 bispecific antibody in Table 7 was added by 3 times dilution series from 17.1 pM for B6-CD3-1, 15.9 pM for B6-CD3-2, and 15.9 pM from B6-CD3-3. Killing rate was calculated in the same manner as in Example 7.

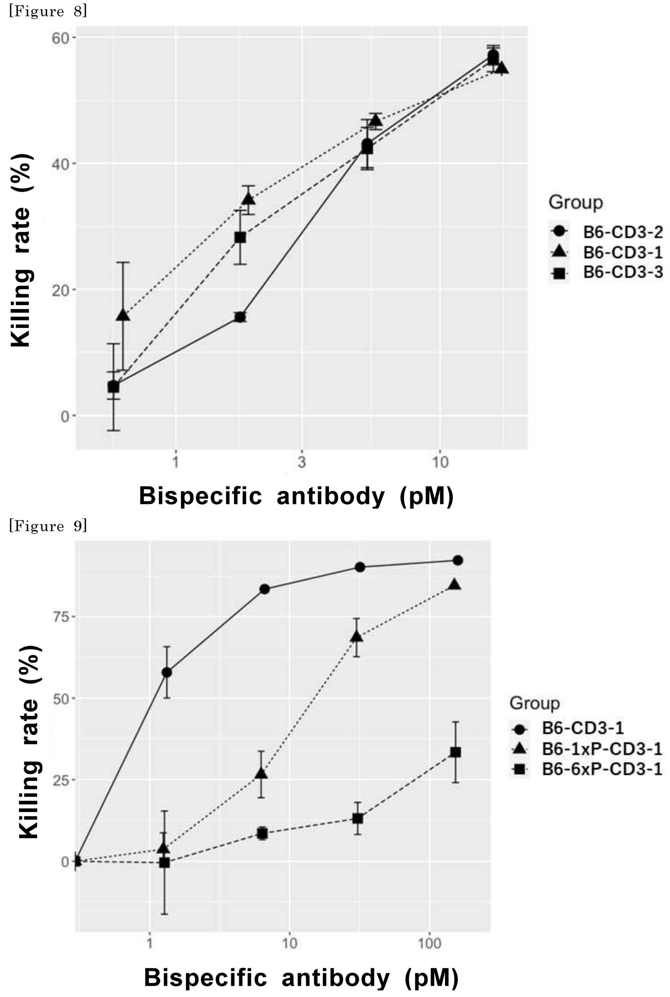

The results are shown in Figure 8. Even when any CCR8-CD3 bispecific antibody was used, concentration-dependent cytotoxic activity was confirmed. EC50 value, 95% confidence interval at that time, and maximum value of killing rate for 6 hours were estimated by Three-variable log-logistic analysis as in Example 7 (Table 8). From the result, a 95% confidence interval for EC50 value of B6-CD3-1 did not overlap with a 95% CI of B6-CD3-2, so it was concluded that there was a significant difference between both EC50 values. From the above, it was found that B6-CD3-1 had the highest cytotoxic activity.

[Table 8]

| Bispecific antibody | EC50 (pM) | Standard error | 95% confidence lower limit (pM) | 95% confidence upper limit (pM) | Maximum value of killing rate |
|---|---|---|---|---|---|
| B6-CD3-1 | 1.37 | 0.24 | 0.83 | 1.9 | 50.7 |
| B6-CD3-2 | 3.55 | 0.52 | 2.41 | 4.69 | 56.7 |
| B6-CD3-3 | 1.62 | 0.19 | 1.22 | 2.03 | 49.3 |

Example 9: Positional optimization of anti-CCR8-scFv region and anti-CD3-scFv region in CCR8-CD3 bispecific antibody (1)

In order to perform relative position optimization of the anti-CCR8-scFv region and the anti-CD3-scFv region in the CCR8-CD3 bispecific antibody, B6-CD3-1 was produced using the sequence of SEQ ID NO: 5 (1$\times$P linker) or SEQ ID NO: 6 (6$\times$P linker) instead of sequence of SEQ ID NO: 3 as an scFv linker (B6-1$\times$P-CD3-1, B6-6$\times$P-CD3-1).

Cytotoxic activity of B6-1$\times$P-CD3-1 and B6-6$\times$P-CD3-1, and B6-CD3-1 was evaluated by a following method. hCCR8/FLuc/Rat-1 ($5\times10^3$/well/$\mu$L) cells used in Exmple 5 as tareget cells and PBMC cells activated in the same manner as in Example 4 as effector cells, were mixed in a 96-well plate at E/T cell ratio 20:1 and the above CCR8-CD3 bispecific antibodies were added by 5 times dilution series from 159 pM for B6-CD3-1, 150 pM for B6-1$\times$P-CD3-1, and 153 pM for B6-6$\times$P-CD3-1 (N=3). After 16 hours, medium was replaced in the same manner as in Example 5 and a luciferase assay was performed. Calculation method of killing rate was the same manner as in Example 7, assuming that a killing rate was 0% when a bispecific antibody was not added.

The results were shown in Figure 9. EC50 value and 95 % confidence interval at that time, and maximum value of killing rate for 6 hours were estimated by Three-variable log-logistic analysis as in Example 7 (Table 9). It was found that B6-CD3-1 had the highest cytotoxic activity so relative positions of CCR8 and CD3 antibody regions were the most excellent. Activity of B6-1×P-CD3-1 and B6-6×P-CD3-1 was decreased. From the above results, it was found that a length between the anti-CCR8-scFv region and the anti-CD3-scFv region in B6-CD3-1 is optimal.

[Table 9]

| Bispecific antibody | EC50 (pM) | Standard error | 95% confidence lower limit (pM) | 95% confidence upper limit (pM) | Maximum value of killing rate |
|---|---|---|---|---|---|
| B6-CD3-1 | 1.21 | 0.3 | 0.56 | 1.9 | 88.6 |
| B6-1xP-CD3-1 | 16.6 | 3.31 | 9.4 | 23.8 | 82.9 |
| B6-6xP-CD3-1 | 36.1 | 11.29 | 11.5 | 60.7 | 33.4 |

Example 10: Cytotoxic activity of CCR8-CD3 bispecific antibody against mature Treg cells

(1) Preparation of mature Treg cells

After PBMCs were separated from human peripheral blood using Ficoll-Paque (Ficoll-Paque PLUS, GEW Healthcare), CD4-positive T cells were purified therefrom using CD4 + T Cell Isolation Kit human (Miltenyi Biotec). Naive Treg cells (CD127 negative, CD4, CD45RA, and CD25-positive fraction) were separated from CD4-positive T cells by FACS Aria (BD Bioscience) using a FITC-labeled anti-human CD4, a PerCP-Cy5.5-labeled anti-human CD45RA, a PECy7-labeled anti-human CD25, and a Bv510-labeled anti-human CD127 antibody. The mature Treg cells were prepared by carrying out expansion culture of naive Treg cells separated according to the protocol of Dynabeads Human Treg Expander (Veritas).

As a result of measuring CCR8 expression on the mature Treg cell surface by a flow cytometer, using an anti-human CCR8 antibody, a CCR8 positive rate in the inducted mature Treg cells was found to be 50%.

(2) Preparation of effector cells

After PBMCs were separated from human peripheral blood using Ficoll-Paque (Ficoll-Paque PLUS, GEW Healthcare), CD8-positive T cells were purified therefrom using CD8 + T Cell Isolation Kit human (Miltenyi Biotec). The cells were used as Fresh effector cells in an assay.

Also, in the presence of IL-2 (Shionogi) 100 U/ml and Dynabeads Human T-Activator CD3/CD28 (Veritas), the separated CD8-positive T cells were cultured at 37°C under 5% $CO_2$ for 10 days in RPMI1640/10 % FCS medium. Thereafter, Dynabeads were removed and cryopreservation was performed by CELLBANKER1 (Zenogen Pharma). The frozen cells were thawed 2 days before the assay, cultured at 37°C under 5% $CO_2$ for 2 days in IL-2 100 U/mL added RPMI1640/10 % FCS medium, and used as Activate effector cells in the assay.

(3) Cytotoxic activity evaluation

The mature Treg cells labeled by Cell Trace Violet (Thermo Fisher) at $1 \times 10^4$/well, and the effector cells at $1 \times 10^5$/well (E/T cell ratio = 10:1) were cultured at 37°C under 5% $CO_2$ for 24 hours or 48 hours in RPMI1640/10 % FCS medium to which IL-2 was added in 96-well plate at 10 U/mL in the presence of the CCR8-CD3 bispecific antibodies (B5-CD3-1, B6-CD3-1, both at final concentrations 530 pM).The cells after culturing were washed once with an HBSS/2% FCS/10 mM HEPES buffer, suspended in 100 $\mu$l of the same buffer, and then stained at 4°C for 30 minutes using LIVE/DEAD (Thermo Fisher Scientific Inc.). The cells were washed twice with the same buffer and the number of the surviving mature Treg cells were measured by a flow cytometer. A CCR8 positive cell rate in the wells containing only the target cells was calculated as 100%

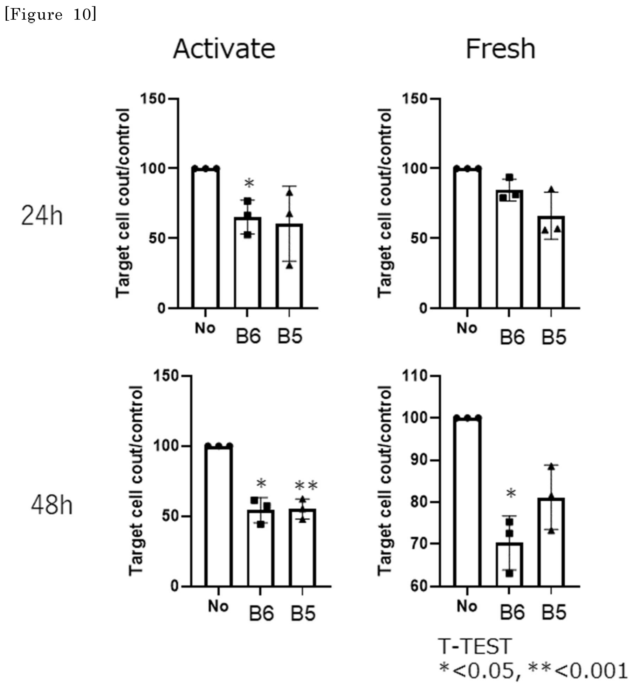

The results are shown in Figure 10. It was confirmed that B5-CD3-1 and B6-CD3-1 had significantly cytotoxic activity as compared to a control (T-test). From the above, it was confirmed that the CCR8-CD3 bispecific antibody exhibits cytotoxic activity against the mature Treg cells expressing CCR8.

Example 11: Cytotoxic activity of CCR8-CD3 bispecific antibody against human tumor-infiltrating Treg cells

(1) Preparation of human tumor-infiltrating cells

Tumor tissues derived from human ovarian cancer patients were dispersed using Tumor Dissociation Kit, human (Miltenyi Biotec) to prepare a cell population containing tumor-infiltrating Treg cells, CD8-positive T cells, and CD4-positive T cells other than Treg cells. For the experiment, the cells immediately after cryopreservation were used.

(2) Measurement of CCR8-positive rate of human tumor-infiltrating Treg cells

For the cell population obtained in the above (1), CCR8-positive rate in tumor-infiltrating Treg cells was measured using flow cytometry.

The cells were suspended in an HBSS/2% FCS/10 mM HEPES buffer to which Human TruStain FcX (BioLegend) and Zombie NIR™ Fixable Viability Kit (BioLegend) were added, and the suspension was allowed to stand at 4°C for 30 minutes. After washing, various antibodies were added, and the mixture was allowed to stand at 4°C for 30 minutes (Bv785-labeled anti-human CD45 antibody, Alexa Flour 700-labeled anti-human CD3 antibody, Bv711-labeled anti-human CD4 antibody, PE-labeled anti-human CCR8 antibody, PE-Dazzle594-labeled anti-human CD56 antibody, Bv510-labeled anti-human CD8 antibody (all manufactured by BioLegend)). After washing, fixation and membrane permeation treatment were performed using eBioscience Foxp3/Transcription Factor Staining Buffer Set (Thermo Fisher Scientific), APC-labeled anti-human Foxp3 (Thermo Fisher Scientific) was added, and the mixture was allowed to stand at 4°C for 30 minutes. After washing, CCR8-positive rate in tumor-infiltrating Treg cells (CD45-, CD3-, CD4-, and Foxp3-positive and Zombie NIR-, CD8-, and CD56-negative fractions) was measured using a flow cytometer.

(3) Evaluation of cytotoxic activity

The ovarian cancer-derived human tumor-infiltrating Treg cells obtained in the above (1) were used as target cells, and the peripheral blood-derived CD8 positive T cells obtained in Example 10 were used as effector cells.

The effector cells and the target cells labeled by CytoTell UltraGreen (AAT BIOQUEST) were seeded on 96-well plates at $1 \times 10^5$ cells/well, and cultured in the presence of the CCR8-CD3 bispecific antibodies (B5-CD3-1, B6-CD3-1, both at final concentrations 530 pM) using RPMI1640/10 % FCS/human IL-2 (100 U/mL). After 24 hours from the start of the evaluation, the cells were collected and suspended in an HBSS/2% FCS/10 mM HEPES buffer to which Human TruStain FcX (BioLegend) and Zombie NIR™ Fixable Viability Kit (BioLegend) were added, and the suspension was allowed to stand at 4°C for 30 minutes. After washing, various antibodies were added, and the mixture was allowed to stand at 4°C for 30 minutes (Bv785-labeled anti-human CD45 antibody, Alexa Flour 700-labeled anti-human CD3 antibody, PE-Cy7-labeled anti-human CD56 antibody, Bv711-labeled anti-human CD4, Bv510-labeled anti-human CD8 antibody (all manufactured by BioLegend, Inc.) and human CCR8 antibody). For a human CCR8 antibody, when B5-CD3-1 was evaluated, APC-labeled CCR8 antibody (BioLegend) was used, when B6-CD3-1 was evaluated, unlabeled CCR8 antibody (ITM) and Alexa647-labeled Donkey Anti-Rat IgG (H+L) were used. After washing, fixation and membrane permeation treatment were performed using eBioscience™ Foxp3/Transcription Factor Staining Buffer Set (Thermo Fisher Scientific Inc.), PE-labeled anti-human Foxp3 (Thermo Fisher Scientific Inc.) was added, and the mixture was allowed to stand at 4°C for 30 minutes. After washing, proportions of CCR8-positive Treg cells (CD45-, CD3-, CD4-Foxp3-, and CCR8-positive and Zombie NIR-, CD8-, and CD56-negative fractions), CD8 positive T cells and CD4-positive Tconv cells in surviving CD45-positive cells were quantified using a flow cytometer.

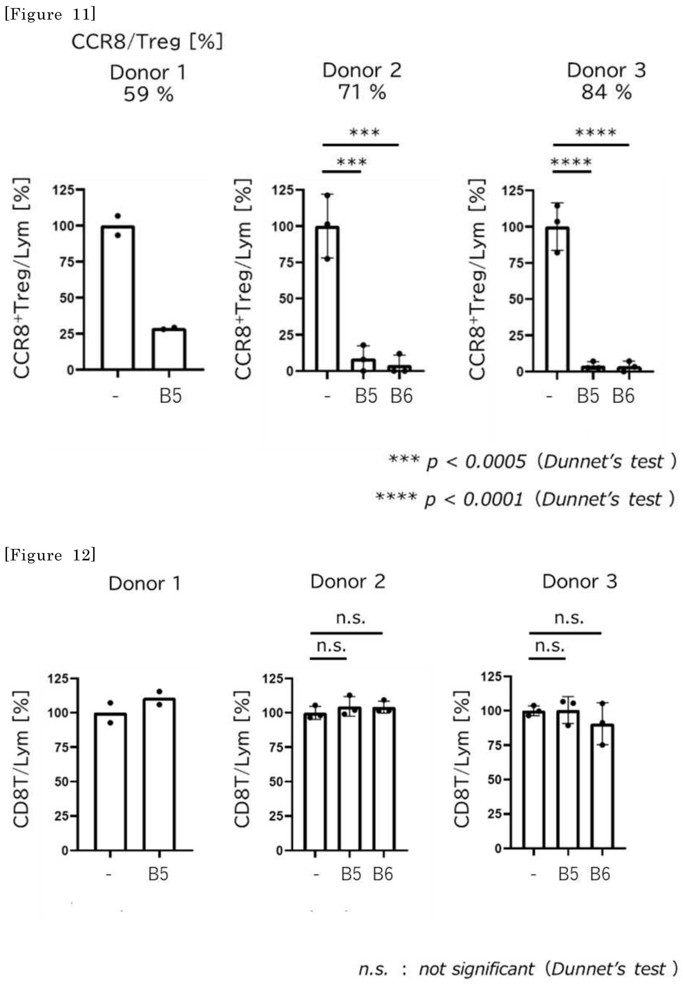

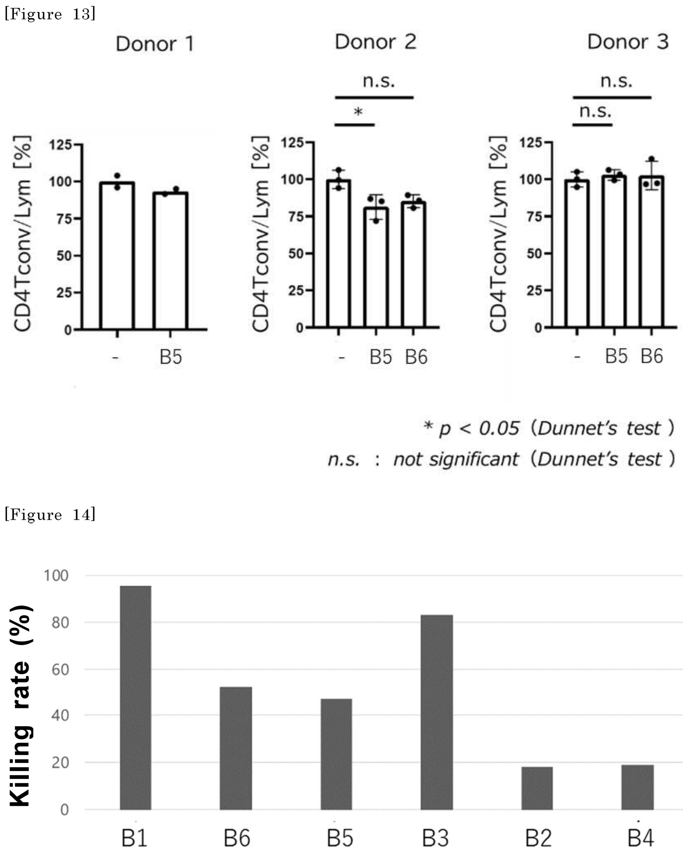

The results were shown in Figure 11. Compared to a control group without the bispecific antibody, it was confirmed that a number of human tumor-infiltrating CCR8 positive Treg cells had significant decreased in the CCR8-CD3 bispecific antibody-added group. Reduction rates for B5-CD3-1 were 71 % in donor 1, 91 % in donor 2, and 96 % in donor3, and reduction rates for B6-CD3-1 were 96 % in donor 2, and 97 % in donor 3 (Dunnet's test, P<0.0005). On the other hand, a significant decrease in CD8-positive T cells was not confirmed in any of the donors (Figure 12). A significant decrease in CD4-positive Tconv cells was not confirmed in donor 1 or donor 3(Figure 13). From the above, it was confirmed that the CCR8-CD3 bispecific antibody had strong cytotoxic activity specifically against tumor-infiltrating Treg cells.

Example 12: Cytotoxic activity of CCR8-CD16 bispecific antibody against human CCR8 expressing cells using human peripheral blood-derived NK cells as effector cells

A cytotoxic activity test was performed using hCCR8/Fluc/Rat-1 cells in Example 5, and CCR8-CD16 bispecific antibodies in Table 10.

[Table 10]

| Bispecific antibody | Anti-CCR8-scFv | Anti-CD16-VHH |
|---|---|---|
| B1-CD16-VHH | αCCR8-B1 | αCD16-VHH |
| B2-CD16-VHH | αCCR8-B2 | αCD16-VHH |
| B3-CD16-VHH | αCCR8-B3 | αCD16-VHH |
| B4-CD16-VHH | αCCR8-B4 | αCD16-VHH |
| B5-CD16-VHH | αCCR8-B5 | αCD16-VHH |
| B6-CD16-VHH | αCCR8-B6 | αCD16-VHH |

hCCR8/Fluc/Rat-1 cells used as target cells were mixed with human peripheral blood-derived NK cells (Biotherapy Institute of Japan) used as effector cells in an assay buffer so that E/T cell ratio was 10:1 (effector cells: $1\times10^5$/well, target cells :$1\times10^4$/well), and were seeded on a 96-well plate. Thereafter, the CCR8-CD16 bispecific antibody was added to each well so as to have a final concentration of 440 pM. A well, to which no bispecific antibody was added, was set up as a control. After the cells and the antibody were added, the cells were centrifuged using a plate centrifuge at 1000 rpm for 2 minutes to allow the cells to settle. After centrifugation, the cells were cultured at 37°C under 5% $CO_2$ for 16 hours. After culture, the washing procedures of adding 100 μL of the assay buffer, centrifuging the cells at 1000 rpm for 2 minutes, and discarding 100 μL of the supernatant, were repeated three times. Finally, 100 μL of the supernatant was discarded and then 50 μL of luciferin was added, and an amount of luminescence in each well was quantified within 30 minutes using a luminometer. Using the amount of luciferase luminescence as an index, cytotoxic activity was calculated using the following calculation formula, assuming that the cells were not damaged (100 % survival) in the well to which no bispecific antibody was added.

{1-(Luminescence value of well to which bispecific antibody was added/ Luminescence value of well to which no bispecific antibody was added)} ×100 (%)

The results are shown in Figure 14. It was confirmed that various CCR8-CD16 bispecific antibodies had cytotoxic activity. Especially, it was confirmed that the CCR8-CD16 bispecific antibodies using B1-CD16-VHH and B3-CD16-VHH had high activity, and it was suggested that B1-CD16-VHH had the highest cytotoxic activity. From the above, it was revealed that the CCR8-CD16 bispecific antibody exhibited cytotoxic activity and B1-CD16-VHH exhibited the highest cytotoxic activity.

Example 13: Cytotoxic activity of CCR8-CD16 bispecific antibody against ATLL cell line

Cytotoxic activity against ATN cells, an ATLL cell line, was evaluated using B1-CD16-VHH as the CCR8-CD16 bispecific antibody.

Calcein-labeled ATN-1 cells were used as target cells, and KHYG-1 cells, human NK cells were used as effector cells. They were mixed in an assay buffer so that E/T cell ratio was 10 : 1, 3 : 1, 1 : 1 (target cells $1\times10^4$/well) and seeded on a 96-well plate. Thereafter, B1-CD16-VHH was added so as to have a final concentration of 4.4 nM. As a control, a well, to which no bispecific antibody was added, was set up. After the cells and the antibody were added, the cells were centrifuged using a plate centrifuge at 1000 rpm for 2 minutes to allow the cells to settle. After centrifugation, the cells were cultured at 37°C under 5% $CO_2$ for 2 hours. After culture, 50 μL of the assay buffer was added, the cell supernatant was separated from the plate. For the separated supernatant, an amount of fluorescence contained in the supernatant was quantified using a fluorescence measuring instrument. A spontaneous release amount (BG) of calcein from ATN cells was determined by measuring the supernatant of s well containing only target cells in the same manner. A total calcein release amount (TOTAL) was determined by adding the assay buffer, separating 70 μL of the cell suspension, and measuring. As a method for calculating killing rate by using calcein release as an index, calculation was performed by the following formula.

{(Fluorescence value of each well - BG fluorescence value)/(TOTAL fluorescence value - BG fluorescence value)} × 100 (%)

BG was subtracted, and assuming that an amount of fluorescence in the supernatant when all cells were killed was 100%, a killing rate (%) was calculated.

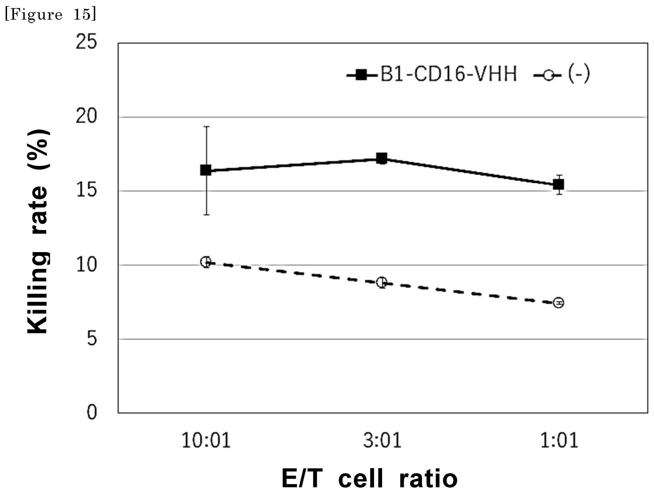

The results are shown in Figure 15. High cytotoxic activity was observed in a well to which B1-CD16-VHH was added at a final concentration of 4.4 nM, as compared to the control. From the above, it was revealed that the CCR8-CD16 bispecific antibody exhibited cytotoxic activity against the ATLL cell line.

Example 14: Cytotoxic activity of CCR8-CD16 bispecific antibody against mature Treg cells

Whether or not cytotoxic activity was exhibited against mature Treg cells induced from human peripheral blood using B1-CD16-VHH as theCCR8-CD16 bispecific antibody was verified. Cytotoxic activity was evaluated in the same manner as in Example 13, using mature Treg cells of Example 10 as target cells and KHYG-1 cells of Example 13 as effector cells.

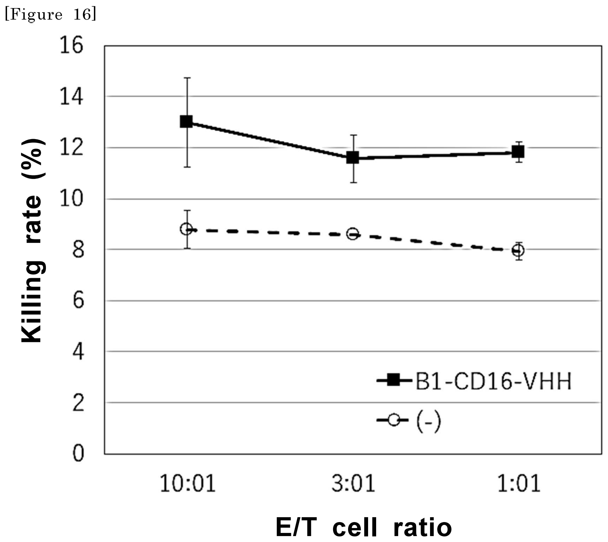

The results are shown in Figure 16. High cytotoxic activity was confirmed by adding B1-CD16-VHH at a final concentration of 4.4 nM, as compared to a control. From the above, it was revealed that the CCR8-CD16 bispecific antibody exhibited cytotoxic activity against the mature Treg cells induced from human peripheral blood.

Example 15: Effect of CCR8 expression or not in target cells, in cytotoxic activity of CCR8-CD16 bispecific antibody

Whether or not cytotoxic activity depended on CCR8 expression was verified using B1-CD16-VHH as the CCR8-CD16 bispecific antibody. Cytotoxic activity was evaluated in the same manner as in Example 13 using CCR8-expressing Rat-1 cells (hCCR8/Rat-1 cells) of Example 4 and a non-CCR8-expressing Rat-1 cell line as target cells, and KHYG-1 cells of Example 13 as effector cells. As a method for calculating killing rate by using calcein release as an index, calculation was performed by the following formula.

{(Fluorescence value of each well - Fluorescence value of well to which no bispecific antibody was added)/(TOTAL fluorescence value - Fluorescence value of well to which no bispecific antibody was added)} $\times$ 100 (%)

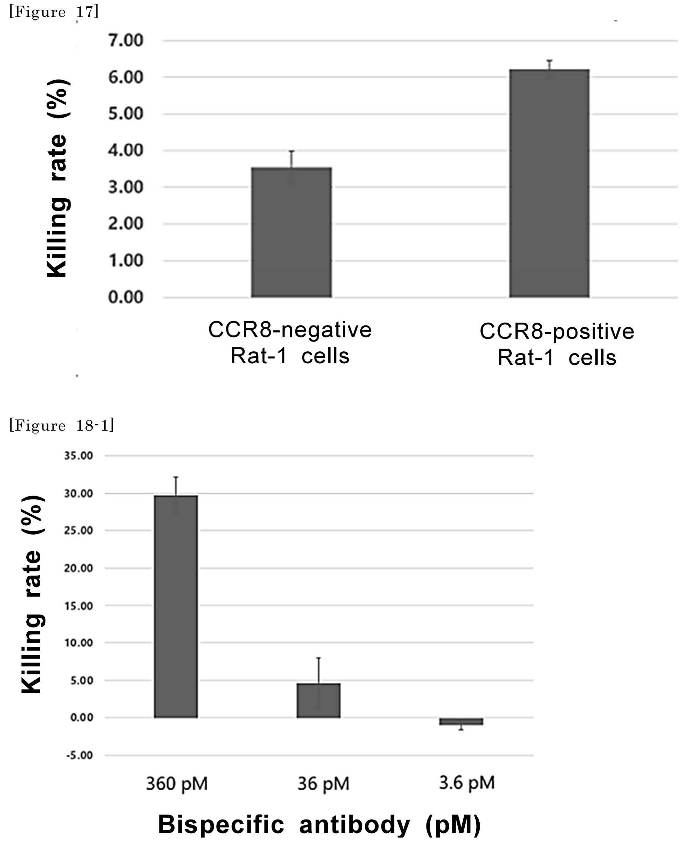

The results are shown in Figure 17. A increase in cytotoxic activity of B1-CD16-VHH against CCR8-expressing Rat-1 cells was observed as compared to CCR8 non-expressing Rat-1 cells.

Example 16: Cytotoxic activity of CCR8-CD3 bispecific antibody comprising anti-mouse CD3-scFv region against human CCR8-expressing cells using CD8-positive T cells derived from mouse spleen as effector cells

(1) Preparation of CD8-positive T cells derived from mouse spleen

Spleen of wild-type mice was removed, passed through a 70 $\mu$m filter and a hemolysis treatment was performed to prepare a cell suspension. Thereafter, CD8-positive T cells were prepared by CD8a+ T Cell Isolation Kit (Miltenyi). The CD8-positive T cells were cultured for 1 day or more and activated by human IL-2 and anti-mouse CD3/28 Dynabeads and then used as effector cells.

(2) Evaluation of cytotoxic activity

Cytotoxic activity was evaluated by a luciferase assay system, using the bispecific antibody (B6-mCD3) having an $\alpha$CCR8-B6-derived anti-CCR8-scFv region and an $\alpha$mCD3-derived anti-CD3-scFv region as a CCR8-CD3 bispecific antibody comprising an anti-mouse CD3-scFv region.

hCCR8/Fluc/Rat-1 cells of Example 5 were used as target cells and mouse spleen-derived CD8 T cells were used as effector cells. These cells were mixed in an assay buffer so that E/T cell ratio was 10:1 (effector cells: $1\times10^5$/well, target cells :$1\times10^4$/well), and were seeded on a 96-well plate. Thereafter, the B6-mCD3 was added to each well so as to have a final concentration of 360 pM, 36 pM, or 3.6 pM. As a control, a well to which no bispecific antibody was added were set up.

After the cells and the antibody were added, the cells were centrifuged using a plate centrifuge at 1000 rpm for 2 minutes to allow the cells to settle. After centrifugation, the cells were cultured at 37°C under 5% $CO_2$ for 16 hours. Thereafter, the washing procedures of adding 100 $\mu$L of an assay buffer, centrifuging the cells at 1000 rpm for 2 minutes, and discarding the supernatant, were repeated three times. Finally, the supernatant was discarded and then 50 $\mu$L of luciferin was added, and an amount of luminescence in each well was quantified using a luminometer.

The results are shown in Figure 18-1. Cytotoxic activity was observed when B1-CD16-VHH was added at 360 nM, as compared to the control. Furthermore, it was also confirmed that cytotoxic activity changed depending on a concentration of the bispecific antibody. From the above, it was revealed that the CCR8-CD3 bispecific antibody comprising the anti-mouse CD3-scFv region exhibited cytotoxic activity.

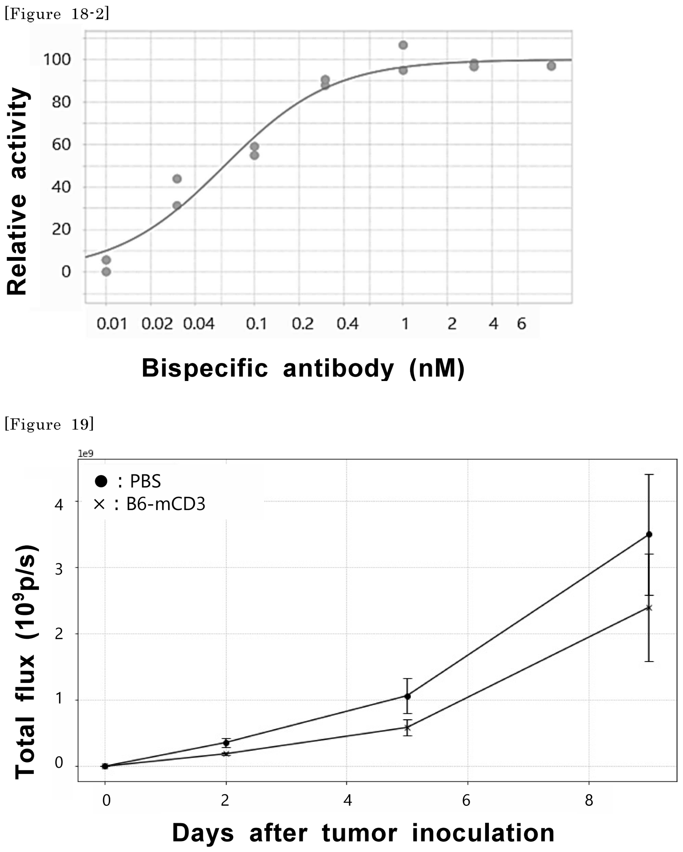

Furthermore, B6-mCD3 was added at final concentrations of 10 pM-10 nM at 50 $\mu$L/well each, the results of cytotoxic activity in the same manner as described above were shown in Figure 18-2. An EC50 was calculated to be 62.3 pM.

Example 17: Anti-tumor activity of CCR8-CD3 bispecific antibody comprising anti-mouse CD3-scFv region in mice inoculated with human CCR8-expressing cells, using mouse spleen-derived CD8-positive T cells as effector cells

hCCR8/Fluc/Rat-1 cells of Example 5 were used as target cells, mouse spleen-derived CD8 T cells were used as effector cells, they were mixed in 100 $\mu$L of PBS so that E/T cell ratio was 2:1 (effector cells :$7\times10^5$, target cells :$3.5\times10^5$), and intraperitoneally administered in NSG mouse (NOD. Cg-PrkdcscidIl2rgtm1Wjl /SzJ, Jackson Laboratory, inc.). Thereafter, 2 $\mu$g of the bispecific antibody (B6-mCD3) was added to each mouse at the time of inoculation, and 1 $\mu$g each after 1 and 2 days from inoculation. Mice to which no bispecific antibody was added were also set up as controls.

After 2, 5, or 9 days from inoculation, proliferation of Rat-1 cells in the abdominal cavity was measured. At the time of measurement, 150 $\mu$L of luciferase substrate (D-Luciferin, 30 mg/mL) was intraperitoneally administered, and luminescence intensity as index of tumor volume in the abdominal cavity was measured after 10 minutes, using IVIS Lumina luminescence detection device.

The results are shown in Figure 19. Anti-tumor activity in the B6-mCD3 administration group was observed as compared to the control group. From the above results, it was revealed that the CCR8-CD3 bispecific antibody comprising as an anti-mouse CD3-scFv region also exhibited activity in vivo.

Example 18: Anti-tumor activity of CCR8-CD3 bispecific antibody in mice inoculated with human CCR8-expressing cells

hCCR8/FLuc/Rat-1 cells ($3.5\times10^6$) of Example 5 which were used as target cells and human PBMCs ($7\times10^6$) activated in the same manner as in Example 4 which were used as effector cells, were mixed in 200 $\mu$L of PBS, and intraperitoneally administered in NSG mouse. After 15 minutes, B6-CD3-1 (1 $\mu$g) dissolved in 200 $\mu$L of PBS or 200 $\mu$L of PBS as a negative control were intraperitoneally administered (N=4). Furthermore, after 2 and 3 days from inoculation, B6-CD3-1 (1 $\mu$g) dissolved in 200 $\mu$L of PBS and the control, PBS, were also intraperitoneally administered.

After 4, 7, 11 and 15 days from inoculation, luminescence intensity was measured in the same manner as in Example 17 by IVIS Lumina luminescence detection device. In measurement, for a body area of region of vertical width of the mouse from the mouth to the base of the tail and horizontal width included the entire mouse, an amount of light released into the same area was calculated as Total Flux (photon/second) and an amount of Photon per second.

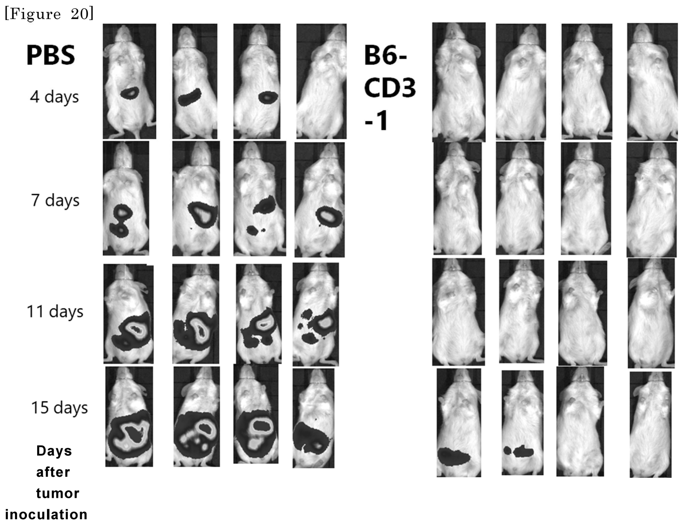

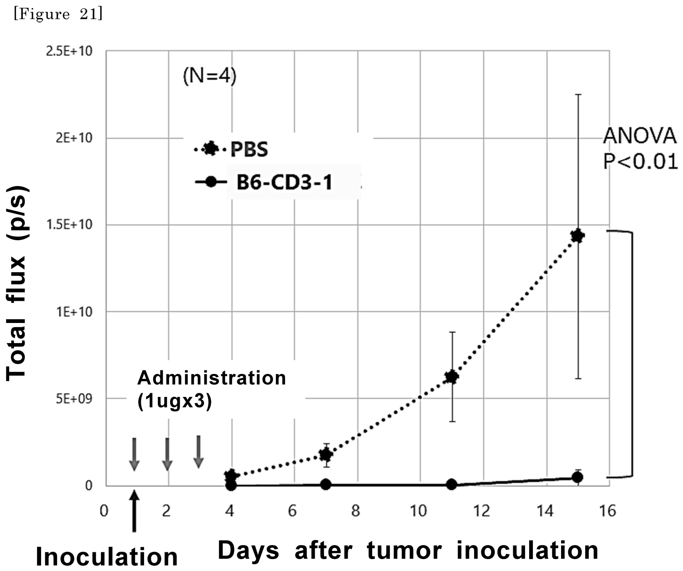

The results are shown in Figure 20 and Figure 21. The graph of Figure 21 is a graph of mean and standard deviation (SD) of luminescence intensity of four mice per group. Measuring until 15 days after inoculation, a significant difference test between groups was tested by ANOVA (two-factor mixed design in analysis of variance), significant differences were observed at a significance level of $P < 0.01$. From the above results, it was showed that the CCR8-CD3 bispecific antibody could kill human CCR8-expressing cells in the abdominal cavity of mice, significant anti-tumor activity was observed in mice.

Example 19: Anti-tumor activity of CCR8-CD3 bispecific antibody against mice inoculated with ATLL cell line

FLuc/ATN-1 cells ($2\times10^6$) of Example 7 which were used as target cells and human PBMCs ($3\times10^6$) activated in the same manner as in Example 4 which were used as effector cells, were mixed in 150 $\mu$L of PBS, 100 $\mu$L of ice-cooled matrigel 354230 (Corning) was added, and intraperitoneally administered in NSG mouse. Thereafter, PBS or B6-CD3-1 (0.75 $\mu$g) dissolved in PBS were administered at 150 $\mu$L. Furthermore, after 2 and 3 days from inoculation, 200 $\mu$L of PBS or 0.75 $\mu$g of B6-CD3-1 dissolved in 200 $\mu$L of PBS, were also intraperitoneally administered.

After 4, 8, and 11 days from inoculation, luminescence intensity was measured in the same manner as in Example 17 by IVIS Lumina luminescence detection device.

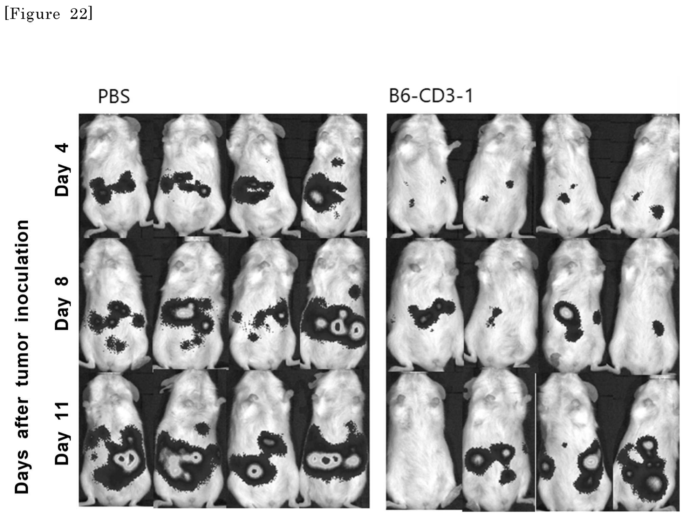

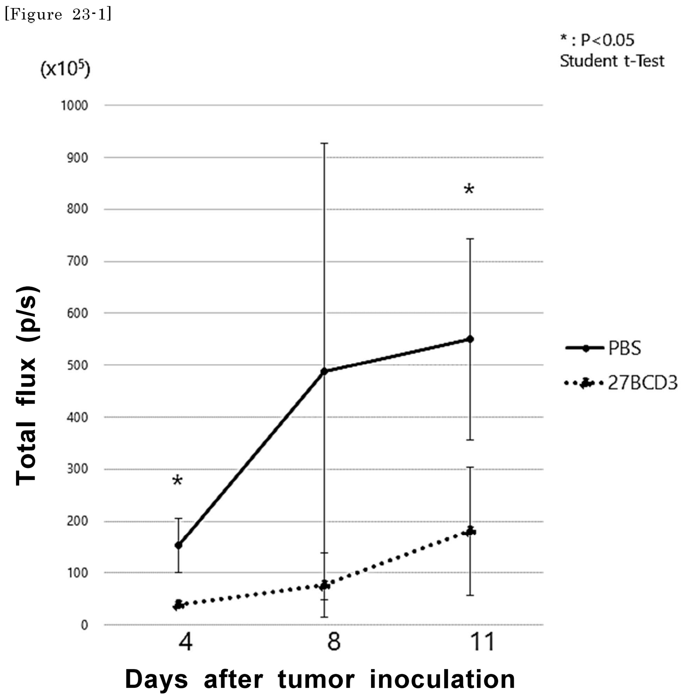

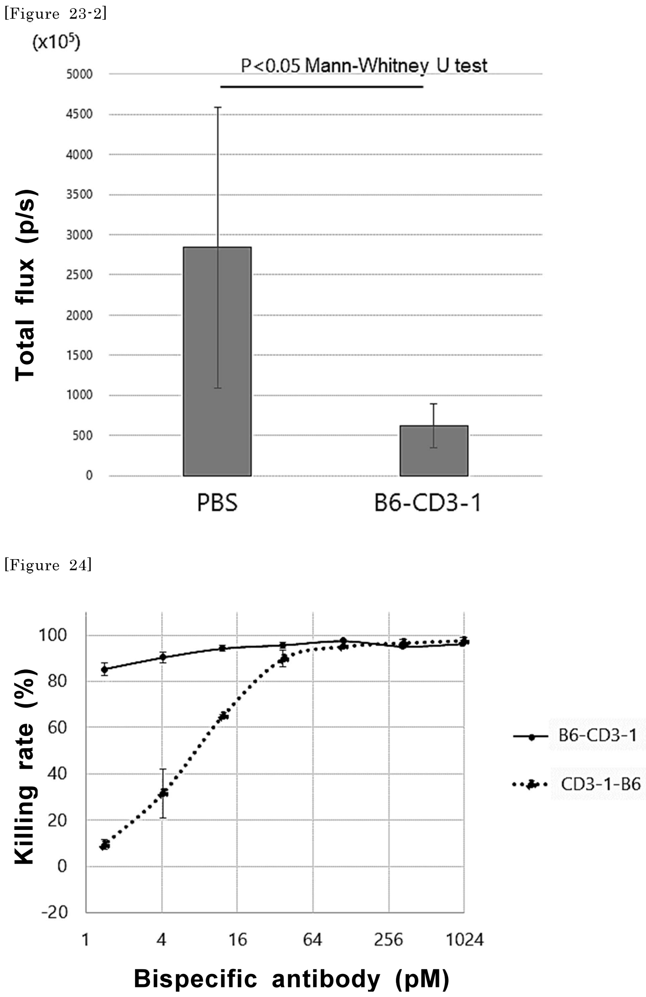

The results are shown in Figure 22 and Figure 23. The graph of Figure 23-1 is a graph of mean and standard deviation (SD) of luminescence intensity of four mice per group. Measuring until 11 days after inoculation, a significant difference test between groups was tested about mean of each measurement day by Student-T, significant differences at two points of 4 and 11 days after inoculation were observed at a significant level of $P < 0.05$. Furthermore, calculating the areas between the graph and X axis for each mouse (AUC, area under curve), the mean and standard deviation (SD) of the areas for each group calculated were in Figure 23-2. A significant difference test about the area between both groups was tested by Mann-Whitney U test. As a result, a significant difference between both groups was observed at a significance level of $P < 0.05$. From the above results, it was showed that the CCR8-CD3 bispecific antibody could kill ATN-1 cells, an ATLL cell line, in the abdominal cavity of mice, significant anti-tumor activity was observed in mice.

Example 20: Cytotoxic activity comparison of CCR8-CD3 bispecific antibody and CD3-CCR8 bispecific antibody

(1) Production of CD3-CCR8 bispecific antibody

The CD3-CCR8 bispecific antibody was produced by swapping the order of an anti-CCR8-scFv region and an anti-CD3-scFv region from the CCR8-CD3 bispecific antibody. Specifically, linking the CD8A signal sequence region (SEQ ID NO: 1), the anti-CCR8-scFv region of αCD3-1(SEQ ID NO: 7), the scFv linker (SEQ ID NO: 3), the anti-CCR8-scFv region of αCCR8-B6 (SEQ ID NO: 44), the histidine tag (SEQ ID NO: 38) in this order from an N-terminal side, the CD3-CCR8 bispecific antibody swapped the order of the anti-CCR8 antibody region and the anti-CD3 region, from B6-CD3-1, was produced (CD3-1-B6). A protein was produced and purified in the same manner as in Example 2.

(2) Cytotoxic activity evaluation

FLuc/ATN-1 cells of Example 7 were used as target cells, human PBMCs activated in the same manner as in Example 4 were used as effector cells. The target cells ($3\times10^3$/well) were seeded on a 96-well round-bottom dish, the effector cells were added so that E/T cell ratio was 2 : 1, and furthermore, the bispecific antibody was added so as to have a final concentration of 1.4 pM from 1000 pM by 3-fold dilution. Final liquid volume per well was 150 μL. An assay buffer was DMEM/4 % FCS. After 16 hours, washing twice with the assay buffer, 50 μL of luciferase substrate was added to the cells, and an amount of luciferase luminescence per well was measured using a luminescence measurement device, after 10 minutes. Killing rate was calculated in the same manner as in Example 7.

The results are shown in Figure 24. An EC50 value of CD3-1-B6 was 7.2 pM $\pm$ 0.96 pM (standard error), 95 % confidence interval at the EC50 value was 5.1 pM to 9.3 pM. On the other hand, an EC50 value of B6-CD3-1 was 0.08 pM $\pm$ 0.16 pM (standard error), 95 % confidence interval at the EC50 value was - 0.27 pM to 0.42 pM. From the above, the order of scFv regions-changed CD3-1-B6 had significantly decreased cytotoxic activity compared to B6-CD3-1 in original orientation and it was revealed that cytotoxic activity was higher in the case of the order the anti-CCR8-scFv region and the anti-CD3-scFv region.

Example 21: Positional optimization of anti-CCR8-scFv region and anti-CD3-scFv region in CCR8-CD3 bispecific antibody (2)

In order to determine whether distance of the scFv regions in the CCR8-CD3 bispecific antibody influenced cytotoxic activity and how long the optimal distance was, B6-L1-CD3-1 into which the ANAS+4xG4S linker (SEQ ID NO: 93) was inserted between the anti-CCR8-scFv region and the anti-CD3-scFv region was produced. A protein was produced and purified in the same manner as in Example 2. One G4S was inserted into B6-CD3-1.

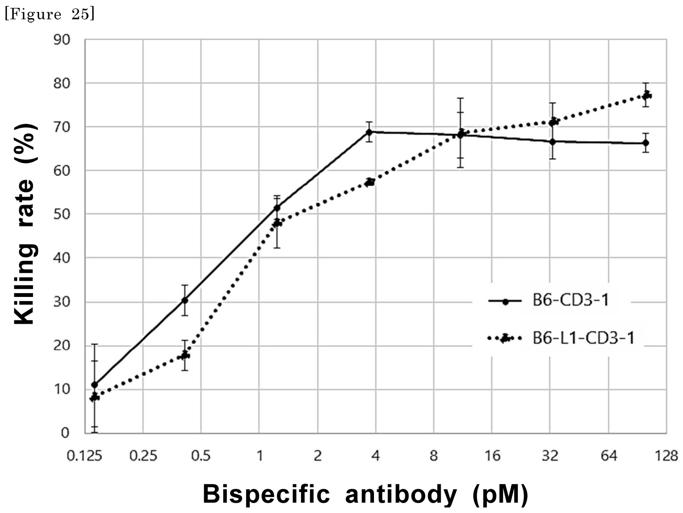

Cytotoxic activity of B6-CD3-1 and B6-L1-CD3-1 was evaluated in the same system as in Example 20, and the results are shown in Figure 25. EC50 values of B6-CD3-1 and B6-L1-CD3-1 were 0.54 pM $\pm$ 0.12 pM (standard error) and 0.91 pM $\pm$ 0.24 pM (standard error) respectively, and B6-CD3-1 had a lower EC50 value.

Example 22: Cytotoxic activity of CCR8-CD3 bispecific antibody with Fc region

The bispecific antibody was produced by binding a single-chain Fc region to a C-terminal side of the CCR8-CD3 bispecific antibody. The Fc region was that of the bispecific antibody described in Patent Document 29, and had the amino sequence of SEQ ID NO: 95. The GSGGGG linker of SEQ ID NO: 94 and the Fc region of SEQ ID NO: 95 were inserted in this order between the anti-CD3-scFv region and a histidine tag of B6-CD3-1, the bispecific antibody was produced (B6-CD3-1-Fc). A protein was produced and purified in the same manner as in Example 2.

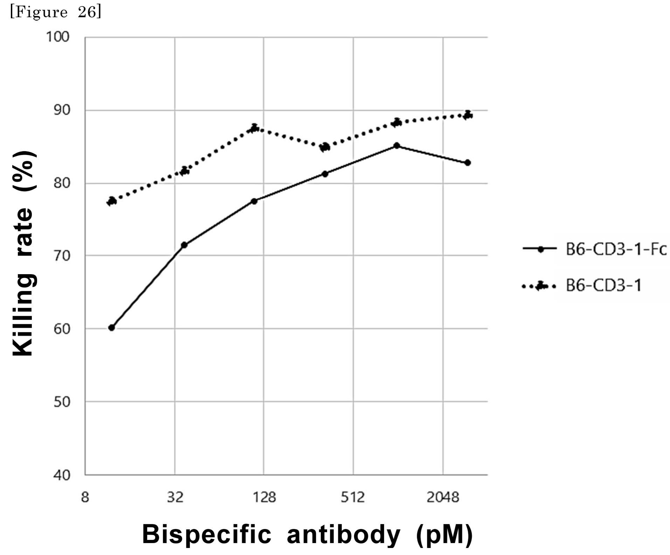

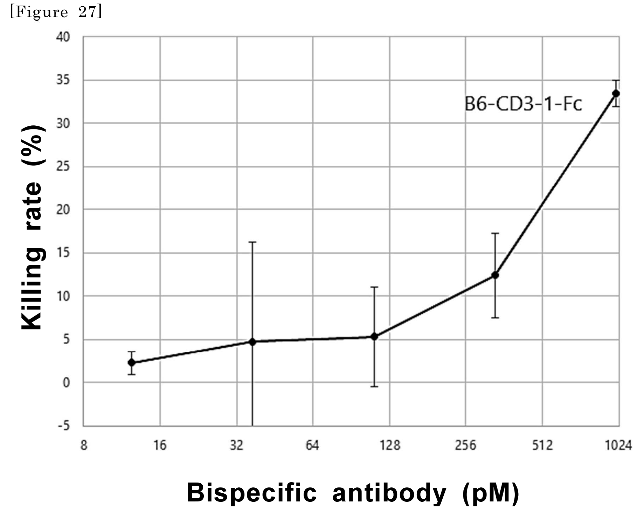

Cytotoxic activity of B6-CD3-1-Fc was evaluated in the same system as in Example 20 using hCCR8/Fluc/Rat-1 cells of Example 5 or FLuc/ATN-1 cells of Example 7 as target cells, and the results are shown in Figure 26 (hCCR8/Fluc/Rat-1 cells) and Figure 27 (FLuc/ATN-1 cells). An EC50 value against hCCR8/Fluc/Rat-1 cells was 9.6 pM $\pm$ 24.6 pM (standard error) and an EC50 value against FLuc/ATN-1 cells was 13.6 pM $\pm$ 52.4 pM (standard error). From the above, it was found that the CCR8-CD3 bispecific antibody, to which the Fc region is added, retains sufficient cytotoxic activity.

Example 23: Comparison of NF-AT promoter induction activity and cytotoxic activity with CCR8-CD3 bispecific antibody based on Patent Document 20

(1) Production of CCR8-CD3 bispecific antibody based on Patent Document 20

The CCR8-CD3 bispecific antibody (563-CCR8-CD3 (SEQ ID NO: 96)) having the amino acid sequence of SEQ ID NO: 96 was produced based on Patent Document 20. Based on Example 4 of Patent Document 20, an anti-CCR8-scFv region was constructed by quoting a variable region sequence of Antibody1 and an anti-CD3-scFv region was constructed by quoting a variable region sequence of I2E antibody. A protein was produced and purified in the same manner as in Example 2.

(2) Evaluation of NF-AT promoter induction activity

Whether the NF-AT promoter of its downstream could be activated by binding 563-CCR8-CD3 to human CCR8 and, also to human CD3 and activating a T cell receptor signal, a complex of CD3, was verified. A system which could detect not only activation between cells in trans but also, activation of CD3 by binding to CCR8 and CD3 on the same cell membrane, what is called activation of NFAT in cis, was constructed. Jurkat cell line (NFAT-Jurkat), a human T cell line, in which a reporter plasmid, into which a luciferase gene was integrated as a reporter gene at downstream of the NF-AT promoter, was stably integrated into a genome, was used (BPS Bioscience inc. NFAT Reporter (Luc)-Jurkat Cell line). A pQEF-CCR8 plasmid (an expression vector obtained by replacing a CMV promoter of pQCXIP with a human EF promoter) expressing human CCR8 was transformed into the cells, drug selection was performed by puromycin at 1 $\mu$g/ml, cells, which were stably expressing human CCR8, were established.

By adding B6-CD3-1 or 563-CCR8-CD3 to the cells at final concentrations of 330 pM and 110 pM, luciferase activity after 16 hours was compared and evaluated (N=1). Assuming that a luciferase activity value was 1 when the bispecific antibody was not added, fold induction was calculated when the bispecific antibody was added.

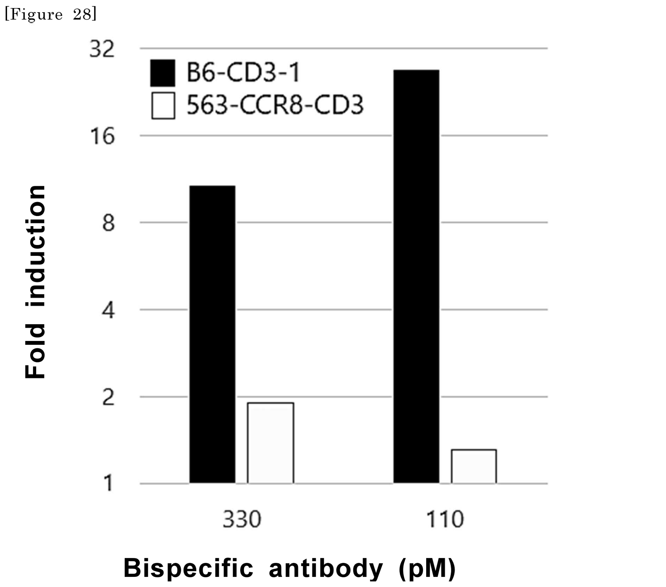

The results are shown in Figure 28. Fold induction of luciferase activity was 10.7-fold and 27-fold at 330 pM and at 110 pM in the case of B6-CD3-1, respectably. On the other hand, fold induction of luciferase activity was 1.9-fold and 1.3-fold in the case of 563-CCR8-CD3, respectably. From the above, it was shown that 563-CCR8-CD3, which was constructed based on Patent Document 20, has lower NFAT promoter inducibility than B6-CD3-1.

(3) Evaluation of cytotoxic activity

Cytotoxic activity of B6-CD3-1 and 563-CCR8-CD3 was evaluated in the same system as in Example 20 using hCCR8/Fluc/Rat-1 cells of Example 5 as target cells. It was performed at 4.1 pM and 1.37 pM of concentrations of the bispecific antibody. E/T cell ratio was 5:1.

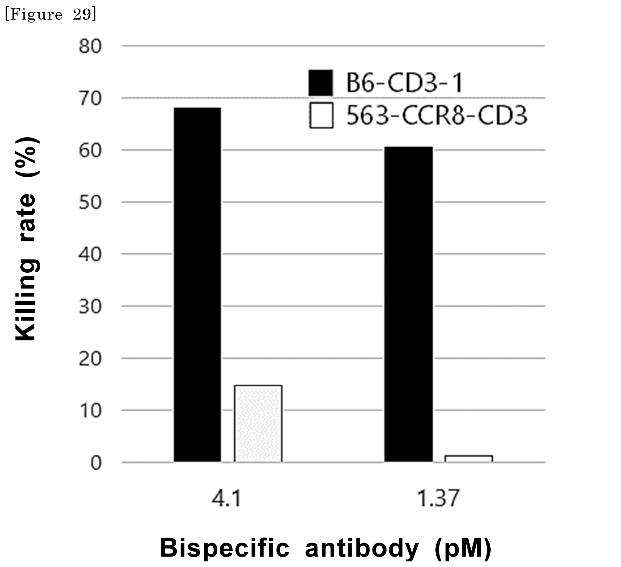

The results are shown in Figure 29. In the case of B6-CD3-1, more than 60% killing activity was exhibited at both concentrations, but, in the case of 563-CCR8-CD3, killing rates were 14.7% at 4.1 pM and 1.33% at 1.37 pM.

Next, Cytotoxic activity was evaluated in the same system as in Example 20, using FLuc/ATN-1 cells as target cells. It was performed by diluting by 3-fold from 32 pM to 0.125 pM of concentrations of the bispecific antibody. E/T cell ratio was 5:1.

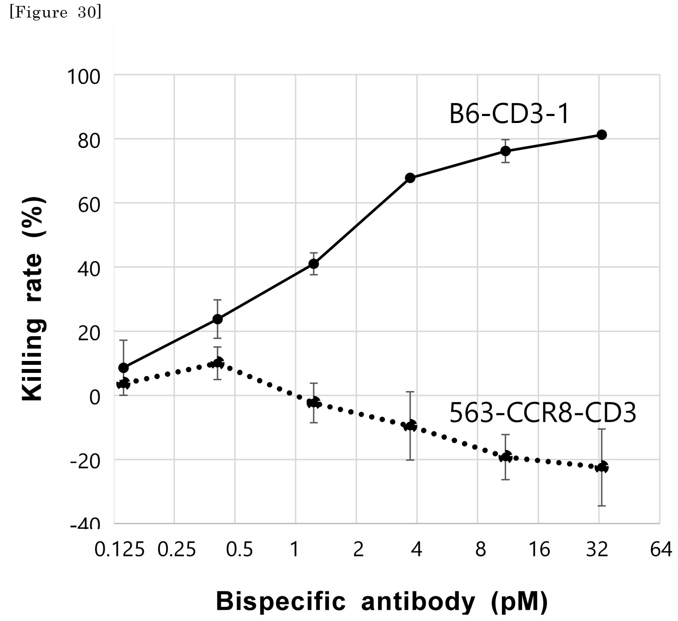

The results are shown in Figure 30. B6-CD3-1 exhibited cytotoxic activity with a EC50 value of 1.6 pM $\pm$ 0.25 pM, whereas 563-CCR8-CD3 had no concentration-dependent killing. As a result of conducting a Student's t-test at all measurement points, B6-CD3-1 exhibited significantly (P<0.05) higher cytotoxic activity than 563-CCR8-CD3 at all measurement points of concentration of more than 0.41 pM. Also, cytotoxic activity of 563-CCR8-CD3 was lower than 10% at any concentrations. From the above, it was shown that 563-CCR8-CD3, which was constructed based on Patent Document 20, has lower cytotoxic activity than B6-CD3-1.

[INDUSTRIAL APPLICABILITY]

A bispecific antibody that recognizes CCR8 as an antigen of the present invention has cytotoxic activity against CCR8-expressing cells. Furthermore, the bispecific antibody that recognizes CCR8 as an antigen of the present invention exhibits an anti-tumor effect. In other words, the bispecific antibody that recognizes CCR8 as an antigen of the present invention is useful for cancer treatment and prevention of cancer.

**Claims**

1. A bispecific antibody that comprises an anti-CCR8-scFv region comprising a light chain variable region and a heavy chain variable region of an anti-CCR8 antibody and a region comprising one or more of a light chain variable region or a heavy chain variable region of an antibody that recognizes a surface marker of an effector cell.

2. The bispecific antibody according to claim 1, wherein the anti-CCR8-scFv region comprises:

    1) a light chain variable region including

        CDR1 having the amino acid sequence of SEQ ID NO: 57,
        CDR2 having the amino acid sequence of SEQ ID NO: 58, and

CDR3 having the amino acid sequence of SEQ ID NO: 59, and
a heavy chain variable region including
CDR1 having the amino acid sequence of SEQ ID NO: 60,
CDR2 having the amino acid sequence of SEQ ID NO: 61, and
CDR3 having the amino acid sequence of SEQ ID NO: 62;

2) a light chain variable region including

CDR1 having the amino acid sequence of SEQ ID NO: 57,
CDR2 having the amino acid sequence of SEQ ID NO: 63, and
CDR3 having the amino acid sequence of SEQ ID NO: 59, and
a heavy chain variable region including
CDR1 having the amino acid sequence of SEQ ID NO: 64,
CDR2 having the amino acid sequence of SEQ ID NO: 61, and
CDR3 having the amino acid sequence of SEQ ID NO: 65;

3) a light chain variable region including

CDR1 having the amino acid sequence of SEQ ID NO: 66,
CDR2 having the amino acid sequence of SEQ ID NO: 67, and
CDR3 having the amino acid sequence of SEQ ID NO: 68, and
a heavy chain variable region including
CDR1 having the amino acid sequence of SEQ ID NO: 69,
CDR2 having the amino acid sequence of SEQ ID NO: 70, and
CDR3 having the amino acid sequence of SEQ ID NO: 71;

4) a light chain variable region including

CDR1 having the amino acid sequence of SEQ ID NO: 72,
CDR2 having the amino acid sequence of SEQ ID NO: 67, and
CDR3 having the amino acid sequence of SEQ ID NO: 73, and
a heavy chain variable region including
CDR1 having the amino acid sequence of SEQ ID NO: 74,
CDR2 having the amino acid sequence of SEQ ID NO: 75, and
CDR3 having the amino acid sequence of SEQ ID NO: 76; or

5) a light chain variable region including

CDR1 having the amino acid sequence of SEQ ID NO: 77,
CDR2 having the amino acid sequence of SEQ ID NO: 78, and
CDR3 having the amino acid sequence of SEQ ID NO: 79, and
a heavy chain variable region including
CDR1 having the amino acid sequence of SEQ ID NO: 80,
CDR2 having the amino acid sequence of SEQ ID NO: 81, and
CDR3 having the amino acid sequence of SEQ ID NO: 82.

3. The bispecific antibody according to claim 1, wherein the anti-CCR8-scFv region comprises:

1) a light chain variable region having the amino acid sequence of SEQ ID NO: 45, and
a heavy chain variable region having the amino acid sequence of SEQ ID NO: 46;
2) a light chain variable region having the amino acid sequence of SEQ ID NO: 47, and
a heavy chain variable region having the amino acid sequence of SEQ ID NO: 48;
3) a light chain variable region having the amino acid sequence of SEQ ID NO: 49, and
a heavy chain variable region having the amino acid sequence of SEQ ID NO: 50;
4) a light chain variable region having the amino acid sequence of SEQ ID NO: 51, and
a heavy chain variable region having the amino acid sequence of SEQ ID NO: 52;
5) a light chain variable region having the amino acid sequence of SEQ ID NO: 53, and
a heavy chain variable region having the amino acid sequence of SEQ ID NO: 54; or

6) a light chain variable region having the amino acid sequence of SEQ ID NO: 55, and
a heavy chain variable region having the amino acid sequence of SEQ ID NO: 56.

4. The bispecific antibody according to any one of claims 1 to 3, wherein the light chain variable region and the heavy chain variable region are linked via the linker sequence of SEQ ID NO: 2 in the anti-CCR8-scFv region.

5. The bispecific antibody according to any one of claims 1 to 4, wherein a CD8A signal sequence region is linked to an N-terminal.

6. The bispecific antibody according to claim 5, wherein the CD8A signal sequence region is present on an N-terminal side of the anti-CCR8-scFv region.

7. The bispecific antibody according to claim 5 or 6, wherein the CD8A signal sequence region that has the amino acid sequence of SEQ ID NO: 1.

8. The bispecific antibody according to any one of claims 1 to 7, wherein the anti-CCR8-scFv region has the amino acid sequence of any one of SEQ ID NOs:39 to 44.

9. The bispecific antibody according to any one of claims 1 to 8, wherein the effector cell is a T cell.

10. The bispecific antibody according to claim 9, wherein the surface marker of the T cell is CD3, comprising an anti-CD3-scFv region that comprises the light chain variable region and the heavy chain variable region of an antibody that recognizes CD3.

11. The bispecific antibody according to claim 10, wherein the anti-CD3-scFv region comprises:

1) a light chain variable region including

CDR1 having the amino acid sequence of SEQ ID NO: 16,
CDR2 having the amino acid sequence of SEQ ID NO: 17, and
CDR3 having the amino acid sequence of SEQ ID NO: 18, and
a heavy chain variable region including
CDR1 having the amino acid sequence of SEQ ID NO: 19,
CDR2 having the amino acid sequence of SEQ ID NO: 20, and
CDR3 having the amino acid sequence of SEQ ID NO: 21;

2) a light chain variable region including

CDR1 having the amino acid sequence of SEQ ID NO: 22,
CDR2 having the amino acid sequence of SEQ ID NO: 23, and
CDR3 having the amino acid sequence of SEQ ID NO: 24, and
a heavy chain variable region including
CDR1 having the amino acid sequence of SEQ ID NO: 25,
CDR2 having the amino acid sequence of SEQ ID NO: 26, and
CDR3 having the amino acid sequence of SEQ ID NO: 27;

3) a light chain variable region including

CDR1 having the amino acid sequence of SEQ ID NO: 28,
CDR2 having the amino acid sequence of SEQ ID NO: 29, and
CDR3 having the amino acid sequence2 of SEQ ID NO: 30, and
a heavy chain variable region including
CDR1 having the amino acid sequence of SEQ ID NO: 31,
CDR2 having the amino acid sequence of SEQ ID NO: 32, and
CDR3 having the amino acid sequence of SEQ ID NO: 33; or

4) a light chain variable region including

CDR1 having the amino acid sequence of SEQ ID NO: 87,
CDR2 having the amino acid sequence of SEQ ID NO: 88, and
CDR3 having the amino acid sequence of SEQ ID NO: 89, and
a heavy chain variable region including
CDR1 having the amino acid sequence of SEQ ID NO: 90,
CDR2 having the amino acid sequence of SEQ ID NO: 91, and
CDR3 having the amino acid sequence of SEQ ID NO: 92.

12. The bispecific antibody according to claim 10, wherein the anti-CD3-scFv region comprises:

1) a light chain variable region having the amino acid sequence of SEQ ID NO: 10, and
a heavy chain variable region having the amino acid sequence of SEQ ID NO: 11;
2) a light chain variable region having the amino acid sequence of SEQ ID NO: 12, and
a heavy chain variable region having the amino acid sequence of SEQ ID NO: 13;
3) a light chain variable region having the amino acid sequence of SEQ ID NO: 14, and
a heavy chain variable region having the amino acid sequence of SEQ ID NO: 15; or
4) a light chain variable region having the amino acid sequence of SEQ ID NO: 85, and
a heavy chain variable region having the amino acid sequence of SEQ ID NO: 86.

13. The bispecific antibody according to claim 10, wherein the anti-CD3-scFv region has the amino acid sequence of SEQ ID NO: 7, 8, 9 or 84.

14. The bispecific antibody according to claim 9, wherein the surface marker of the T cell is CD3, comprising an anti-CD3-VHH region comprises a heavy chain variable region of a VHH antibody that recognizes CD3.

15. The bispecific antibody according to any one of claims 1 to 8, wherein the effector cell is an NK cell.

16. The bispecific antibody according to claim 15, wherein the surface marker of the NK cell is CD16, comprising an anti-CD16-scFv region that comprises a light chain variable region and a heavy chain variable region of an antibody that recognizes CD16.

17. The bispecific antibody according to claim 15, wherein the surface marker of the NK cell is CD16, comprising an anti-CD16-VHH region that comprises a heavy chain variable region of a VHH antibody that recognizes CD16.

18. The bispecific antibody according to claim 17, wherein the anti-CD16-VHH region comprises a heavy chain variable region including

CDR1 having the amino acid sequence of SEQ ID NO: 35,
CDR2 having the amino acid sequence of SEQ ID NO: 36, and
CDR3 having the amino acid sequence of SEQ ID NO: 37.

19. The bispecific antibody according to claim 17, wherein the anti-CD16-VHH region has the amino acid sequence of SEQ ID NO: 34.

20. The bispecific antibody according to any one of claims 1 to 19, wherein the anti-CCR8-scFv region comprising the light chain variable region and the heavy chain variable region of the anti-CCR8 antibody and the region comprising one or more of a light chain variable region or the heavy chain variable region of the antibody that recognizes the surface maker of the effector cell are linked via a scFv linker sequence of any one of SEQ ID NOs: 3 to 6.

21. The bispecific antibody according to any one of claims 1 to 20, wherein a histidine tag having the amino acid sequence of SEQ ID NO: 38 is linked to a C-terminal.

22. The bispecific antibody according to any one of claims 1 to 21 having a Fc region.

23. A pharmaceutical composition comprising the bispecific antibody according to any one of claims 1 to 22.

24. The pharmaceutical composition according to claim 23, for use in treating cancer.

**25.** The pharmaceutical composition according to claim 24 having an effect of depleting tumor-infiltrating Treg cells expressing CCR8.

**26.** The pharmaceutical composition according to claim 24, for use in treating cancer that is not expressing CCR8 in tumor cells.

**27.** The pharmaceutical composition according to claim 24, for use in treating cancer expressing CCR8 in tumor cells.

**28.** A polynucleotide encoding an amino acid sequence of the bispecific antibody according to any one of claims 1 to 22.

**29.** An expression vector comprising the polynucleotide according to claim 28.

[Figure 1]

IL2_PBMC

○—hCCR8/Rat-1
●—Rat-1

**Effector cell**     +       +       −
**Bispecific antibody**   +       −       +

CD3/28 beads+IL2_PBMC

○—hCCR8/Rat-1
●—Rat-1

**Effector cell**     +       +       −
**Bispecific antibody**   +       −       +

[Figure 2]

[Figure 3]

[Figure 4]

FoxP3 positive 99.5%
Median
Iso 33
FoxP3 2447

FoxP3 positive 90%
Median
Iso 289
FoxP3 1143

FoxP3 positive 90.5%
Median
Iso -410
FoxP3 1365

[Figure 5]

[Figure 6]

[Figure 7]

[Figure 8]

[Figure 9]

[Figure 10]

[Figure 11]

CCR8/Treg [%]

Donor 1
59 %

Donor 2
71 %

Donor 3
84 %

*** p < 0.0005 (Dunnet's test)

**** p < 0.0001 (Dunnet's test)

[Figure 12]

Donor 1

Donor 2

Donor 3

n.s. : not significant (Dunnet's test)

[Figure 13]

$* p < 0.05$ (Dunnet's test)
n.s. : not significant (Dunnet's test)

[Figure 14]

[Figure 15]

[Figure 16]

[Figure 17]

[Figure 18-1]

[Figure 18-2]

[Figure 19]

[Figure 20]

[Figure 21]

[Figure 22]

[Figure 23-1]

[Figure 23-2]

[Figure 24]

[Figure 25]

[Figure 26]

[Figure 27]

[Figure 28]

[Figure 29]

[Figure 30]

## INTERNATIONAL SEARCH REPORT

| International application No. |
| --- |
| **PCT/JP2024/019695** |

**A. CLASSIFICATION OF SUBJECT MATTER**

*C07K 16/28*(2006.01)i; *A61K 39/395*(2006.01)i; *A61P 35/00*(2006.01)i; *A61P 35/02*(2006.01)i; *A61P 43/00*(2006.01)i; *C07K 16/46*(2006.01)i; *C12N 15/13*(2006.01)i; *C12N 15/63*(2006.01)i

FI: C07K16/28 ZNA; C12N15/63 Z; A61P35/00; A61P43/00 105; A61K39/395 N; A61K39/395 T; A61P35/02; C07K16/46; C12N15/13

According to International Patent Classification (IPC) or to both national classification and IPC

**B. FIELDS SEARCHED**

Minimum documentation searched (classification system followed by classification symbols)

C07K16/28; A61K39/395; A61P35/00; A61P35/02; A61P43/00; C07K16/46; C12N15/13; C12N15/63

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
| --- | --- | --- |
| X | WO 2022/256559 A1 (AMGEN RES MUNICH GMBH) 08 December 2022 (2022-12-08) claims, examples | 1, 9, 10, 22-29 |
| Y | claims, examples | 1-29 |
| Y | JP 2022-505158 A (REGENTS OF THE UNIVERSITY OF MINNESOTA) 14 January 2022 (2022-01-14) claims, examples, paragraph [0026] | 15-29 |
| Y | WO 2022/211046 A1 (SHIONOGI & CO., LTD.) 06 October 2022 (2022-10-06) claims, examples, paragraph [0017] | 1-29 |

☐ Further documents are listed in the continuation of Box C.     ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **08 August 2024** | **20 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/019695** |

**Box No. I          Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/019695**

| Patent document cited in search report | | | Publication date (day/month/year) | Patent family member(s) | | | Publication date (day/month/year) |
|---|---|---|---|---|---|---|---|
| WO | 2022/256559 | A1 | 08 December 2022 | EP 4347654 A1 claims, examples | | | |
| JP | 2022-505158 | A | 14 January 2022 | WO 2020/081841 A1 claims, examples, paragraph [0045] | | | |
| WO | 2022/211046 | A1 | 06 October 2022 | EP 4317439 A1 claims, examples | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10087259 B
- WO 2018181425 A
- WO 2018112032 A
- WO 2020138489 A
- WO 2021142002 A
- WO 2021152186 A
- WO 2021163064 A
- WO 2021178749 A
- WO 2021194942 A
- WO 2021260208 A
- WO 2021260209 A
- WO 2021260210 A
- WO 2022042690 A
- WO 2022078277 A
- WO 2022081718 A
- WO 2022117569 A
- WO 2022136647 A
- WO 2022136649 A
- WO 2022136650 A
- WO 2022256563 A
- WO 2022268192 A
- WO 2023288241 A
- WO 2023010054 A
- WO 2023020621 A
- WO 2022200303 A
- WO 2013131010 A
- WO 9954440 A
- WO 2016182751 A
- WO 2017134158 A
- WO 2020081841 A
- WO 2023098888 A
- WO 2023116880 A
- WO 2023137466 A
- WO 2023174396 A
- WO 2023193732 A
- WO 2023206350 A
- WO 2023208182 A
- WO 2024008110 A
- WO 2024040216 A
- WO 2024059909 A
- WO 2024062019 A
- WO 2024062072 A
- WO 2024062076 A
- WO 2024062082 A
- WO 2024076514 A
- WO 2024086684 A
- WO 2024088346 A
- WO 2024094003 A
- WO 2022211046 A

**Non-patent literature cited in the description**

- *J. Immunol.*, 1996, vol. 157 (7), 2759-63
- *J. Biol. Chem.*, 1997, vol. 272 (28), 17251-4
- *Cancer Res.*, 2016, vol. 76 (4)
- *Immunity*, 2016, vol. 45, 1122-1134
- *Immunity*, 2016, vol. 45, 1135-1147
- *Proc. Natl. Acad. Sci. USA*, 2018, vol. 115 (45), 10672-10681
- **DANIEL O VILLARREAL et al.** Targeting of CCR8 induces anti-tumor activity as a monotherapy that is further enhanced in combination with a Listeria-based immunotherapy. *ASCO*, 2018, https://www.advaxis.com/wp-content/uploads/2018/04/Keysto-nePosterCCR8-combo-posterFINAL.pdf
- *Cancer Res.*, 2018, vol. 78 (18), 5340-5348
- *Leuk. Lymphoma.*, 2006, vol. 47 (10), 2163-73
- *Appl. Immunohistochem. Mol. Morphol.*, 2015, vol. 23 (8), 580-589
- *Modern Pathology*, 2013, vol. 26, 182-194
- *Exp. Hematol.*, 2021, vol. 10, 56
- Molecular Cloning, A Laboratory Manua. Cold Spring Harbor Laboratory Press, 2012
- *Current Protocols Essential Laboratory Techniques, Current Protocols*, 2012
- **SATO, K. et al.** *Cancer Res.*, 1993, vol. 53, 851
- **FERNANDES et al.** *J. Biol. Chem.*, 2012, vol. 287 (16), 13324-13335